(19) Europäisches Patentamt
European Patent Office
Office européen des brevets

(11) Numéro de publication: **0 331 144 B1**

(12) **FASCICULE DE BREVET EUROPEEN**

(45) Date de publication de fascicule du brevet: **09.06.93**

(51) Int. Cl.$^5$: **C07C 217/84**, C07C 323/36, C07C 323/09, C07C 205/37, A61K 7/13

(21) Numéro de dépôt: **89103577.6**

(22) Date de dépôt: **01.03.89**

# (54) Para-aminophénols 3-substitués, leur procédé de préparation, leur utilisation pour la teinture des fibres kératiniques et les composés intermédiaires utilisés dans le procédé de préparation.

(30) Priorité: **03.03.88 FR 8802713**

(43) Date de publication de la demande:
**06.09.89 Bulletin 89/36**

(45) Mention de la délivrance du brevet:
**09.06.93 Bulletin 93/23**

(84) Etats contractants désignés:
**AT BE CH DE ES FR GB GR IT LI NL SE**

(56) Documents cités:
**LU-A- 56 491**

(73) Titulaire: **L'OREAL**
**14, Rue Royale**
**F-75008 Paris(FR)**

(72) Inventeur: **Junino, Alex**
**16, rue Docteur Bergonié**
**F-93180 Livry Gargan(FR)**
Inventeur: **Genet, Alain**
**9, rue des Coquelicots**
**F-93600 Aulnay sous Bois(FR)**
Inventeur: **Lang, Gérard**
**44, Avenue Lacour**
**F-95210 Saint Gratien(FR)**

(74) Mandataire: **Casalonga, Axel et al**
**BUREAU D.A. CASALONGA - JOSSE Morassistrasse 8**
**W-8000 München 5 (DE)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.6/3.3.1)

## Description

L'invention a pour objet des para-aminophénols 3-substitués, leur procédé de préparation et leur utilisation dans des compositions tinctoriales pour la teinture des fibres kératiniques et en particulier des cheveux humains, ces compositions tinctoriales étant utilisées pour la coloration dite coloration d'oxydation ou teinture permanente.

Ce procédé de coloration permet de teindre les cheveux blancs dans un grand nombre de nuances.

La teinture des fibres kératiniques par la coloration dite d'oxydation est connue. Cette teinture utilise des précurseurs de colorants d'oxydation appelés également base d'oxydation qui sont incolores, mais au contact d'un oxydant, développent dans les fibres kératiniques une coloration durable. On connait comme précurseurs de colorants d'oxydation les para-phénylènediamines, les ortho-phénylènediamines, les para-aminophénols, les ortho-aminophénols, substitués ou non substitués. Ces précurseurs de colorants d'oxydation peuvent être mélangés avec un ou plusieurs composés appelés "coupleurs". Ces coupleurs sont généralement choisis parmi les méta-diamines, les méta-aminophénols, les méta-diphénols et les phénols.

L'invention a pour objet les compositions tinctoriales pour fibres kératiniques et en particulier pour cheveux humains contenant dans un véhicule aqueux un ou plusieurs précurseurs de colorants d'oxydation, de formule (I) :

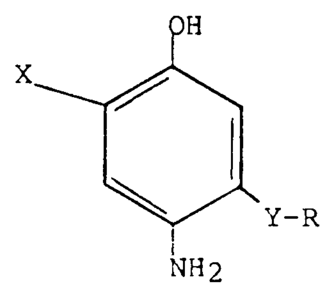

(I)

dans laquelle X représente un atome d'hydrogène ou d'halogène, Y représente un atome d'oxygène ou de soufre et R représente un radical alkyle en $C_1$-$C_4$, hydroxyalkyle en $C_1$-$C_4$, polyhydroxyalkyle en $C_3$-$C_6$ ou amino-alkyle en $C_1$-$C_4$, avec la condition que lorsque X désigne un atome d'hydrogène et Y désigne un atome d'oxygène, R n'est pas méthyle; ou un sel d'un ou plusieurs composés de formule (I). Les sels sont généralement des chlorhydrates ou des sulfates.

On utilise de préférence les composés de formule (I) dans laquelle X, Y et R ont les significations mentionnées ci-dessus, avec la condition que lorsque X désigne un atome d'hydrogène et Y un atome d'oxygène, R ne représente pas un radical alkyle.

Les compositions tinctoriales contenant un ou plusieurs composés de formule (I) permettent de conférer aux cheveux des couleurs stables aux lavages, à la lumière et aux intempéries. En outre, les para-aminophénols de formule (I) bénéficient d'une bonne innocuité.

L'invention a également pour objet les composés nouveaux que constituent les p-aminophénols 3-substitués de formule (I) dans laquelle X représente un atome d'hydrogène ou d'halogène, Y représente un atome d'oxygène ou de soufre, R représente un radical alkyle en $C_1$-$C_4$, hydroxyalkyle en $C_1$-$C_4$, polyhydroxyalkyle en $C_3$-$C_6$ ou amino-alkyle en $C_1$-$C_4$ avec la condition que :

(i) lorsque X représente un atome d'hydrogène et Y représente un atome d'oxygène, R n'est pas méthyle ou éthyle;

(ii) lorsque X représente un atome d'hydrogène et Y représente un atome de soufre, R n'est pas méthyle; et leurs sels.

Les composés préférés sont ceux de formule (I) dans laquelle X, Y et R ont les significations mentionnées ci-dessus, avec la condition que lorsque X représente un atome d'hydrogène, R est différent d'un radical alkyle.

L'invention vise également le procédé de préparation des composés de formule (I). Ces composés sont préparés en deux étapes à partir du nitrophénol substitué de formule (III), dans laquelle X désigne hydrogène ou halogène; Z désigne un atome d'halogène selon le schéma réactionnel 1 ci-dessous.

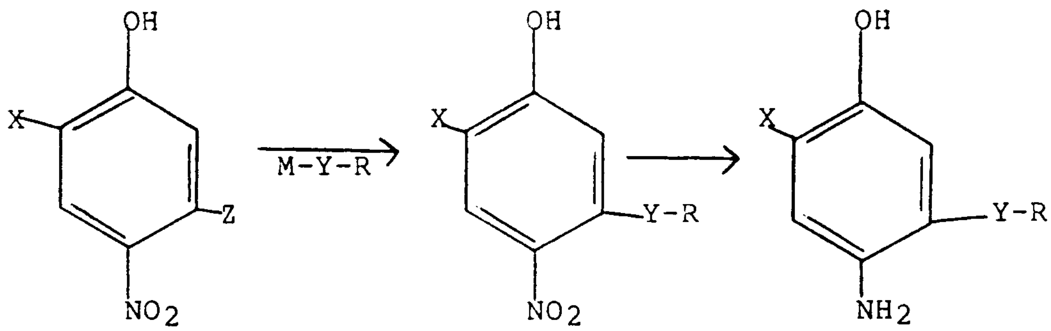

(III) (II) (I)

Schéma réactionnel 1

Le composé (I) est obtenu par réduction du composé (II), composé pour lequel X, Y et R ont les significations ci-dessus définies. Parmi les méthodes de réductions classiques, on peut citer la réduction par l'hydrosulfite de sodium en milieu alcalin à une température inférieure à 70 °C, ou bien une réduction catalytique en milieu hydro-alcoolique sous pression d'hydrogène en présence d'un catalyseur tel que le palladium sur charbon ou le nickel.

Le composé (II) est préparé par action d'un alcoolate ou d'un thiolate de formule (IV) :

M - Y - R (IV)

dans laquelle M représente un métal alcalin ou alcalino-terreux et Y et R ont les significations ci-dessus définies.

L'alcoolate ou le thiolate (IV) peut être avantageusement préparé in situ selon le schéma réactionnel 2 :

$MOH + H\text{-}Y\text{-}R \rightarrow M\text{-}Y\text{-}R + H_2O$

Schéma réactionnel 2

le composé H-Y-R pouvant également servir de solvant.

Parmi les solvants que l'on peut utiliser pour la préparation des composés (II), on peut citer, outre le composé H-Y-R, le dioxane, le N,N-diméthylformamide, la N-méthylpyrrolidone, utilisés seuls ou en mélange; généralement la température de la réaction est inférieure à 120 °C.

Le composé (I) pour lequel X = H, Y = O et R = $\beta$-hydroxyéthyle peut être également préparé selon le schéma réactionnel 3, en deux étapes :

a) condensation du sel de diazonium de l'acide p-sulfanilique sur le 3-($\beta$-hydroxyéthoxy)phénol;

b) réduction du composé azoïque obtenu par l'hydrosulfite de sodium :

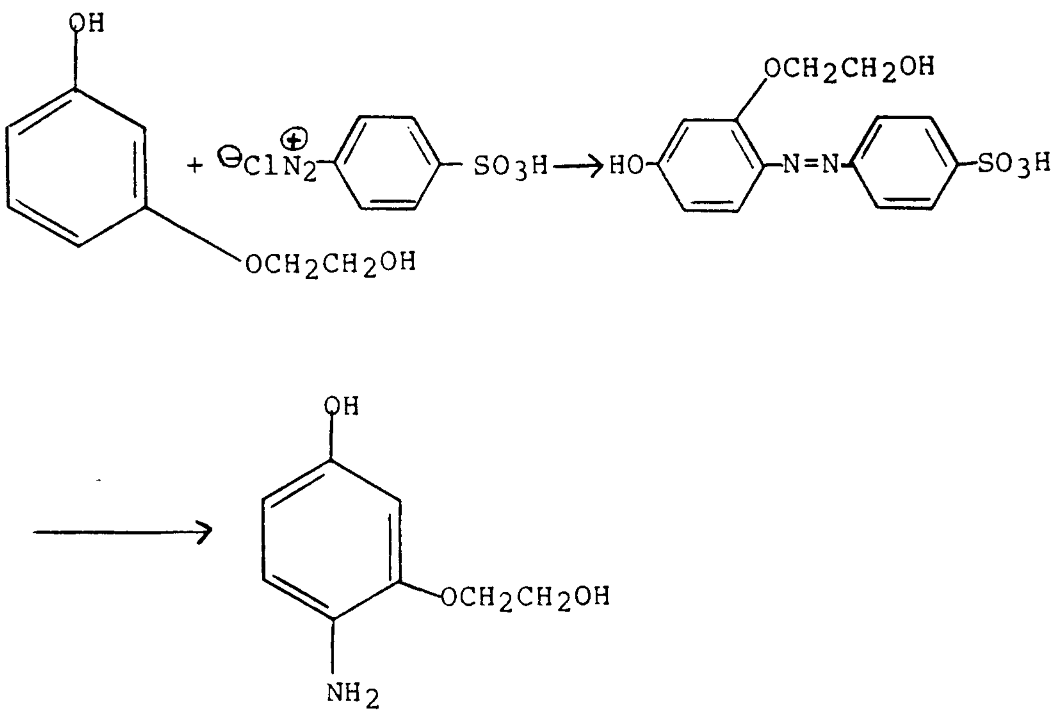

Schéma réactionnel 3

L'invention a également pour objet les nouveaux para-nitrophénols de formule (II) utilisés comme composés intermédiaires dans la préparation des composés de formule (I).

En particulier, l'invention a pour objet des p-nitrophénols substitués de formule :

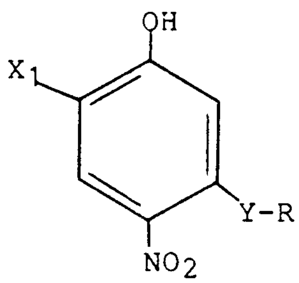

(II)

dans laquelle :

(i) Y représente un atome d'oxygène ou de soufre, $X_1$ représente un atome d'hydrogène et R représente un radical amino-alkyle en $C_{1-4}$ ou polyhydroxyalkyle en $C_3$-$C_6$, ou

(ii) Y représente un atome d'oxygène, $X_1$ représente un atome d'halogène et R représente un radical alkyle en $C_{2-4}$, un radical hydroxyalkyle en $C_{1-4}$, polyhydroxyalkyle en $C_3$-$C_6$ ou un radical amino-alkyle en $C_{1-4}$, ou

(iii) Y représente un atome de soufre, $X_1$ représente un atome d'halogène et R représente un radical alkyle en $C_{1-4}$, hydroxyalkyle en $C_{1-4}$, polyhydroxyalkyle en $C_3$-$C_6$ ou amino-alkyle en $C_{1-4}$;

et leurs sels, et en particulier les chlorhydrates et les sulfates.

D'autres objets de l'invention apparaîtront à la lecture de la description, y compris les exemples.

L'invention est illustrée par les exemples de préparation non limitatifs ci-après.

EXEMPLES DE PREPARATION

EXEMPLE 1

Préparation de l'amino-4 (β-hydroxyéthoxy)-3 phénol

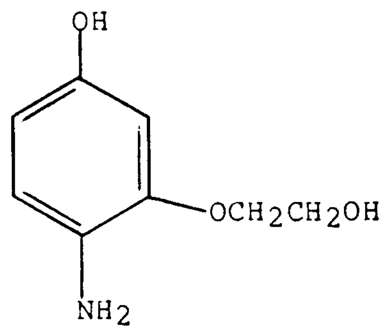

1er stade :
Préparation du (β-hydroxyéthoxy)-3 nitro-4 phénol

A une solution de 0,3 mole de KOH dans 22,5 ml d'éthylèneglycol, chauffée au bain marie bouillant, on ajoute par portions 0,1 mole (17,3 g) de chloro-3 nitro-4 phénol puis 10 ml de dioxane. On maintient le chauffage 11 heures.

Le phénate du produit attendu est essoré après refroidissement du milieu réactionnel. Il est alors lavé au dioxane puis à l'éthanol et enfin dissous dans 100 ml d'eau. Le produit attendu précipite par acidification. Recristallisé de l'isopropanol, il fond à 167 ° C.

L'analyse du produit obtenu donne les résultats suivants :

| Analyse | Calculé pour $C_8H_9NO_5$ | Trouvé |
|---|---|---|
| %C | 48,24 | 48,19 |
| %H | 4,56 | 4,63 |
| %N | 7,03 | 6,96 |
| %O | 40,17 | 39,98 |

2ème stade :
Préparation de l'amino-4 (β-hydroxyéthoxy)-3 phénol

A la solution constituée par 19 g de NaOH en pastilles et par le (β-hydroxyéthoxy)-3 nitro-4 phénol, préparé au stade précédent, dans 160 ml d'eau, on ajoute sous agitation et par portions 59 g d'hydrosulfite de sodium en maintenant la température du milieu réactionnel inférieure à 70 ° C.

A la fin de l'addition, après refroidissement et neutralisation par l'acide sulfurique, le produit attendu précipite. Il fond à 165 ° C. Il est transformé en chlorhydrate par traitement avec l'acide chlorhydrique concentré.

Le chlorhydrate du produit attendu est recristallisé d'une solution hydroalcoolique d'acide chlorhydrique.

L'analyse du chlorhydrate obtenu donne les résultats suivants :

| Analyse | Calculé pour $C_8H_{12}NO_3Cl$ | Trouvé |
|---|---|---|
| %C | 46,72 | 46,57 |
| %H | 5,88 | 5,80 |
| %N | 6,81 | 6,80 |
| %O | 23,34 | 23,58 |
| %Cl | 17,24 | 17,18 |

EXEMPLE 2

Préparation de l'amino-4 (β-hydroxyéthylthio)-3 phénol

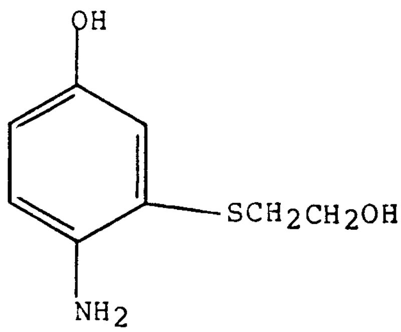

1er stade :
Préparation du (β-hydroxyéthylthio)-3 nitro-4 phénol

A une solution de 0,4 mole de KOH dans 35 ml de thioéthanol, chauffée à 40 °C, on ajoute 0,2 mole (34,7 g) de chloro-3 nitro-4 phénol dans 20 ml de N-méthylpyrrolidone.

On chauffe 1 heure au bain marie bouillant. Le milieu réactionnel est dilué par 400 ml d'eau glacée. Après neutralisation par l'acide acétique, le produit attendu cristallise.

Recristallisé de l'acétate d'éthyle, il fond à 161 °C.

L'analyse du produit obtenu donne les résultats suivants :

| Analyse | Calculé pour $C_8H_9NO_4S$ | Trouvé |
|---|---|---|
| %C | 44,66 | 44,60 |
| %H | 4,22 | 4,32 |
| %N | 6,51 | 6,42 |
| %O | 29,74 | 29,58 |
| %S | 14,88 | 14,77 |

2ème stade :
Préparation de l'amino-4 (β-hydroxyéthylthio)-3 phénol

A la solution constituée par 38 g NaOH en pastilles et par le (β-hydroxyéthylthio)-3 nitro-4 phénol, préparé au stade précédent, dans 320 ml d'eau, on ajoute sous agitation et par portions, 118 g d'hydrosulfite de sodium en maintenant la température inférieure à 70 °C.

A la fin de l'addition, après refroidissement et neutralisation par l'acide acétique, le produit attendu précipite. Il est recristallisé de l'acétate d'éthyle ou de l'acétonitrile. Il fond à 85 °C.

L'analyse du produit obtenu donne les résultats suivant :

| Analyse | Calculé pour $C_8H_{11}NO_2S$ | Trouvé |
|---|---|---|
| %C | 51,88 | 52,01 |
| %H | 5,99 | 5,91 |
| %N | 7,56 | 7,38 |
| %O | 17,28 | 17,44 |
| %S | 17,28 | 17,26 |

EXEMPLE 3

Préparation du chlorhydrate de l'amino-4 chloro-6 ($\beta$-hydroxyéthoxy)-3 phénol

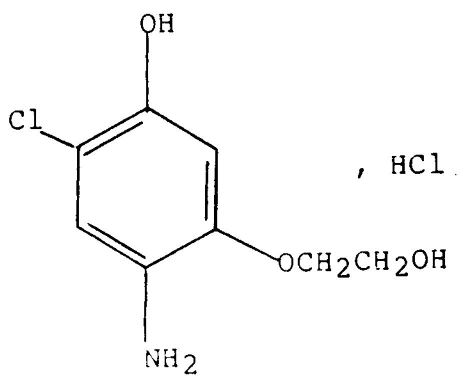

1er stade :
Préparation du chloro-6 ($\beta$-hydroxyéthoxy)-3nitro-4 phénol

A une solution de 0,36 mole de KOH dans 80 ml d'éthylèneglycol chauffée à 50 °C, on ajoute 0,12 mole (25 g) de dichloro-3,6 nitro-4 phénol (préparé selon Fries, Annalen der Chemie 454, page 247 (1927)). On chauffe 2 heures 30 à 120 °C.

Après refroidissement, on essore le phénate du produit attendu qui sera utilisé directement au stade suivant.

Le produit attendu est préparé par acidification à l'acide acétique d'une solution aqueuse du phénate ci-dessus.

Recristallisé de l'acétonitrile, il fond à 159 °C.

L'analyse du produit recristallisé donne les résultats suivants :

| Analyse | Calculé pour $C_8H_8NO_5Cl$ | Trouvé |
|---|---|---|
| %C | 41,13 | 41,21 |
| %H | 3,45 | 3,49 |
| %N | 6,00 | 5,90 |
| %O | 34,24 | 33,98 |
| %Cl | 15,18 | 15,06 |

2ème stade :
Préparation du chlorhydrate d'amino-4 chloro-6 ($\beta$-hydroxyéthoxy)-3 phénol

A une solution de 32 g de phénate de chloro-6 ($\beta$-hydroxyéthoxy)-3 nitro-4 phénol et de 75 ml de NaOH 10 N dans 250 ml d'eau, on ajoute par portions, sous agitation, 70 g d'hydrosulfite de sodium en maintenant la température au voisinage de 60-65 °C. Après refroidissement, on précipite le produit par neutralisation du milieu réactionnel par l'acide acétique. Le chlorhydrate est obtenu par dissolution dans l'acide chlorhydrique à chaud et précipitation par refroidissement.

L'analyse du produit obtenu donne les résultats suivants :

| Analyse | Calculé pour $C_8H_{11}NO_3Cl_2$ | Trouvé |
|---|---|---|
| %C | 40,02 | 39,83 |
| %H | 4,62 | 4,66 |
| %N | 5,83 | 5,69 |
| %O | 19,99 | 19,71 |
| %Cl | 29,53 | 29,62 |

EXEMPLE 4

Préparation de l'amino-4 chloro-6 méthylthio-3 phénol

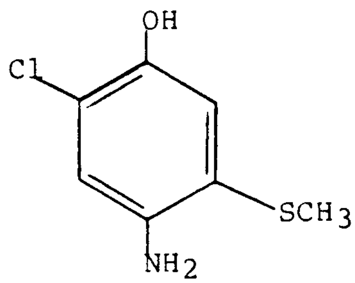

1er stade :
Préparation du chloro-6 méthylthio-3 nitro-4 phénol, N-méthylpyrrolidone (le colorant cristallise avec une mole de N-méthyl pyrrolidone)

A la suspension de 0,35 mole (25 g) de thiométhylate de sodium dans 50 ml de N-méthylpyrrolidone, on ajoute en une demi-heure, par portions, en maintenant la température inférieure à 45 ° C, 0,17 mole (35,4 g) de dichloro-3,6 nitro-4 phénol. On chauffe 30 minutes à 45 ° C. Par dilution du milieu réactionnel par 400 ml d'eau glacée, puis acidification par l'acide acétique, le produit attendu cristallise avec la N-méthylpyrrolidone.

Recristallisé de l'alcool, il fond à 189 ° C;

L'analyse du produit obtenu donne les résultats suivants :

| Analyse | Calculé pour $C_{12}H_{15}N_2O_4SCl$ | Trouvé |
|---|---|---|
| %C | 45,21 | 45,11 |
| %H | 4,74 | 4,81 |
| %N | 8,79 | 8,97 |
| %O | 20,07 | 19,97 |
| %S | 10,06 | 9,88 |
| %Cl | 11,12 | 10,97 |

2ème stade :
Préparation de l'amino-4 chloro-6 méthylthio-3 phénol

A la solution de 32 g de NaOH en pastilles dans 270 ml d'eau, on ajoute le chloro-6 méthylthio-3 nitro-4 phénol préparé au stade précédent, puis rapidement, sans dépasser la température de 70 ° C, 100 g d'hydrosulfite de sodium. On agite 15 minutes supplémentaires après la fin de l'addition.

Par refroidissement, puis neutralisation par l'acide acétique, on précipite le produit attendu du milieu réactionnel.

Recristallisé de l'éthanol à 96°, il fond à 188°C.
L'analyse du produit obtenu donne les résultats suivants :

| Analyse | Calculé pour $C_7H_8NOSCl$ | Trouvé |
|---|---|---|
| %C | 44,33 | 44,38 |
| %H | 4,25 | 4,41 |
| %N | 7,38 | 7,37 |
| %O | 8,43 | 8,68 |
| %Cl | 18,69 | 19,00 |
| %S | 16,91 | 16,78 |

EXEMPLE 5

Préparation de l'hydrate du dichlorhydrate de l'amino-4 chloro-6 (β-aminoéthylthio)-3 phénol

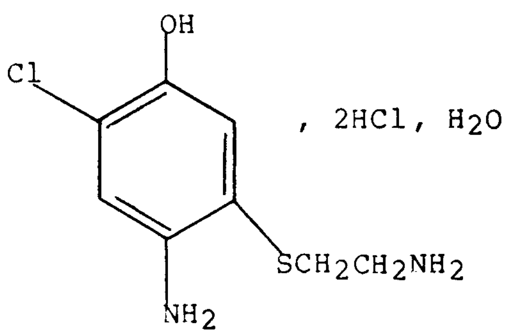

1er stade :
Préparation du chlorhydrate de (β-aminoéthylthio)-3 chloro-6 nitro-4 phénol

A température ambiante, on ajoute 0,04 mole (8,3 g) de dichloro-3,6 nitro-4 phénol à 0,1 mole de chlorhydrate d'amino-2 éthanethiol et 0,14 mole de carbonate de potassium dans 40 ml de N-méthylpyrrolidone. On chauffe 5 heures au bain marie bouillant le milieu réactionnel ci-dessus après avoir ajouté 0,2 mole de KOH en pastilles.

Par addition de 300 g de glace, puis acidification entre pH 1 et 2, on précipite le produit de départ qui n'a pas réagi. Le produit attendu est obtenu par précipitation, par addition d'acide acétique au milieu réactionnel préalablement alcalinisé par une solution de NaOH 10 N. Il est recristallisé d'un mélange hydroalcoolique contenant de l'acide chlorhydrique.

L'analyse du produit obtenu donne les résultats suivants :

| Analyse | Calculé pour $C_8H_{10}N_2O_3SCl_2$ | Trouvé |
|---|---|---|
| %C | 33,70 | 33,77 |
| %H | 3,53 | 3,63 |
| %N | 9,82 | 9,66 |
| %O | 16,83 | 16,81 |
| %S | 11,24 | 11,14 |
| %Cl | 24,87 | 24,92 |

2ème stade :
Préparation de l'hydrate du dichlorhydrate d'amino-4 chloro-6 (β-aminoéthylthio)-3 phénol

A la solution de l'hydrate du chlorhydrate de (β-aminoéthylthio)-3 chloro-6 nitro-4 phénol et de 4,6 g de NaOH en pastilles dans 32 ml d'eau, on ajoute rapidement par portions 12 g d'hydrosulfite de sodium en maintenant la température inférieure à 70 °C. Le milieu réactionnel est, après refroidissement, neutralisé par de l'acide acétique. Par traitement de la gomme ainsi obtenue par l'acide chlorhydrique concentré, on obtient le produit attendu. Il est recristallisé d'un mélange d'acide chlorhydrique et d'éthanol.

L'analyse du produit obtenu donne les résultats suivants :

| Analyse | Calculé pour $C_8H_{15}Cl_3N_2O_2S$ | Trouvé |
|---|---|---|
| %C | 31,04 | 30,99 |
| %H | 4,88 | 4,90 |
| %N | 9,04 | 8,89 |
| %O | 10,34 | 10,42 |
| %S | 10,36 | 10,25 |
| %Cl | 34,36 | 34,21 |

Les compositions tinctoriales pour fibres kératiniques et en particulier pour cheveux humains, selon l'invention, contiennent, dans un véhicule aqueux, au moins un composé de formule (I).

Les composés de formule (I) sont utilisés dans les compositions de l'invention à des concentrations comprises entre 0,02 et 6% et de préférence comprises entre 0,15 et 5% en poids, par rapport au poids total de la composition.

Les compositions de l'invention peuvent contenir également d'autres précurseurs de colorants par oxydation.

Parmi ces précurseurs de colorants par oxydation, il faut citer les para-phénylènediamines, les para-aminophénols, les ortho-phénylènediamines et les ortho-aminophénols.

Parmi les para-phénylènediamines, il faut citer plus particulièrement :
la para-phénylènediamine,
la para-toluylènediamine,
la diméthyl-2,6 para-phénylènediamine,
la diméthyl-2,6 méthoxy-3 para-phénylènediamine,
la N-(β-méthoxyéthyl)para-phénylènediamine,
la N-[β-(β'-hydroxyéthoxy)éthyl] amino-4 aniline,
la N,N-(β-hydroxyéthyl)amino-4 aniline,
la N,N-(éthyl, carbamylméthyl)amino-4 aniline,
ainsi que leurs sels.

Parmi les para-aminophénols, il faut citer plus particulièrement :
le para-aminophénol,
le méthyl-2 amino-4 phénol,
le chloro-2 amino-4 phénol,
le diméthyl-2,6 amino-4 phénol,
le diméthyl-2,3 amino-4 phénol,
le diméthyl-2,5 amino-4 phénol,
l'hydroxyméthyl-2 amino-4 phénol,
le β-hydroxyéthyl-2 amino-4 phénol,
ainsi que leurs sels.

Parmi les ortho-phénylènediamines et les ortho-aminophénols pouvant être substitués sur le noyau ou sur les fonctions amines, il faut citer en particulier l'amino-1 hydroxy-2 benzène, le méthyl-6 hydroxy-1 amino-2 benzène, le méthyl-4 amino-1 hydroxy-2 benzène.

Les compositions tinctoriales faisant l'objet de la présente invention contiennent généralement en association avec les composés (I) et éventuellement avec des para-phénylènediamines ou avec d'autres para-aminophénols, des coupleurs qui donnent par couplage oxydatif avec les bases d'oxydation des indo-anilines, des indamines ou des indophénols, diversement nuancés qui contribuent à modifier et à enrichir de reflets les colorations "de fond" conférées aux cheveux par les produits de condensation des bases d'oxydation sur elles-mêmes.

Parmi les coupleurs, on peut citer en particulier les méta-diphénols, les méta-aminophénols, les méta-phénylènediamines, les méta-acylaminophénols, les méta-uréidophénols, les méta-carbalcoxyaminophénols, l'$\alpha$-naphtol, les coupleurs possédant un groupe méthylène actif tels que les composés $\beta$-cétoniques et les pyrazolones.

Parmi les méta-diphénols on peut citer :
la résorcine,
la méthyl-2 résorcine,
la méthyl-5 résorcine,
le dihydroxy-2,4 phénoxyéthanol,
le monométhyléther de résorcine,
le dihydroxy-2,4 anisole.

Parmi les méta-aminophénols, on peut citer :
le méta-aminophénol,
le méthyl-2 amino-5 phénol,
le méthyl-2 [N-($\beta$-hydroxyéthyl)amino]-5 phénol,
le méthyl-2 [N-($\beta$-mésylaminoéthyl)amino]-5 phénol,
le diméthyl-2,6 amino-3 phénol,
l'hydroxy-6 benzomorpholine,
et leurs sels.

Parmi les métaphénylènediamines, on peut citer :
la méta-phénylènediamine,
le diamino-2,4 phénoxyéthanol,
le diméthoxy-2,4 diamino-1,3 benzène,
le triméthoxy-1,3,5 diamino-2,4 benzène,
le diamino-2,4 anisole,
l'amino-6 benzomorpholine,
le [N-($\beta$-hydroxyéthyl)amino]-2 amino-4 phénoxyéthanol,
le [N-($\beta$-hydroxyéthyl)amino]-4 amino-2 phénoxyéthanol,
l'amino-2 [N-($\beta$-hydroxyéthyl)amino]-4 anisole,
le di-($\beta$-hydroxyéthoxy)-4,5 diamino-1,3 benzène,
le $\beta$-hydroxyéthoxy-1 diamino-2,4 benzène,
et leurs sels.

Parmi les autres coupleurs utilisables dans les compositions tinctoriales de l'invention, il faut citer plus particulièrement :
le méthylènedioxy-3,4 phénol,
le méthylènedioxy-3,4 aniline,
le bromo-2 méthylènedioxy 4,5-phénol,
le chloro-2 méthylènedioxy-4,5 phénol.
le méthoxy-2 méthylènedioxy-4,5 aniline.

Le poids total des précurseurs de colorants d'oxydation et des coupleurs utilisés dans les compositions tinctoriales selon l'invention est de préférence de 0,1 à 7% en poids du poids total de la composition tinctoriale.

Les compositions tinctoriales selon l'invention peuvent également renfermer des colorants directs tels que des colorants azoïques, anthraquinoniques et les dérivés nitrés de la série benzénique, qui permettent de nuancer ou d'enrichir en reflets les colorations apportées par les précurseurs de colorants d'oxydation et/ou les coupleurs.

Le pH de la composition tinctoriale est généralement compris entre 8 et 11 et de préférence entre 9 et 11. Ce pH est ajusté à la valeur désirée à l'aide d'un agent alcalinisant tel que l'ammoniaque, les carbonates alcalins, les alcanolamines telles que la mono-, la di- ou la triéthanolamine.

Les compositions tinctoriales conformes à l'invention contiennent dans leur forme de réalisation préférée des agents tensio-actifs anioniques, cationiques, non-ioniques, amphotères ou leurs mélanges. Ces agents tensio-actifs sont présents dans les compositions conformes à l'invention dans des proportions comprises entre 0,5 et 40% en poids, et de préférence entre 2 et 30% en poids par rapport au poids total de la composition.

Ces compositions peuvent également contenir des solvants organiques pour solubiliser les composés qui ne seraient pas suffisamment solubles dans l'eau. Parmi ces solvants, on peut mentionner à titre d'exemple, les alcools en $C_1$-$C_8$ tels que l'éthanol, l'isopropanol, l'alcool benzylique et l'alcool phényléthylique; le glycérol; les glycols ou éthers de glycol comme le butoxy-2 éthanol, l'éthylèneglycol, le propylèneglycol, le monoéthyléther et le monométhyléther du diéthylèneglycol, ainsi que les produits analogues et leurs mélanges. Les solvants sont présents de préférence dans une proportion comprise entre 1 et 40% en poids, et en particulier entre 2 et 30% en poids par rapport au poids total de la composition.

Les agents épaississants que l'on peut ajouter dans les compositions conformes à l'invention sont pris notamment dans le groupe formé par l'alginate de sodium, la gomme arabique, les dérivés de la cellulose tels que la méthylcellulose, l'hydroxyéthylcellulose, l'hydroxypropylcellulose, l'hydroxyméthylcellulose, la carboxyméthylcellulose, les polymères d'acide acrylique, la gomme de xanthane. On peut également utiliser des agents épaississants minéraux tels que la bentonite. Ces agents épaississants sont présents de préférence en des proportions comprises entre 0,1 et 5% en poids, et en particulier entre 0,5 et 3% en poids du poids total de la composition.

Les compositions peuvent contenir des agents antioxydants choisis en particulier parmi le sulfite de sodium, l'acide thioglycolique, le bisulfite de sodium, l'acide ascorbique et l'hydroquinone. Ces agents antioxydants sont présents dans la composition dans des proportions comprises entre 0,05 et 1,5% en poids du poids total de la composition.

D'autres adjuvants utilisables conformément à l'invention sont par exemple des agents de pénétration, des agents séquestrants, des tampons et des parfums.

Les compositions tinctoriales conformes à l'invention peuvent se présenter sous des formes diverses telles que sous forme de liquides, de crèmes, de gels ou sous toute autre forme appropriée, pour réaliser une teinture des fibres kératiniques et notamment des cheveux humains. Elles peuvent également être conditionnées en flacons aérosols en présence d'un agent propulseur.

Les compositions tinctoriales selon l'invention sont utilisées dans un procédé de teinture des cheveux mettant en oeuvre la révélation par un oxydant.

Conformément à ce procédé, on mélange au moment de l'emploi la composition tinctoriale décrite ci-dessus avec une solution oxydante, en une quantité suffisante pour oxyder les précurseurs de colorants d'oxydation, ensuite on applique sur les cheveux le mélange obtenu.

La solution oxydante contient en solution aqueuse des agents d'oxydation choisis dans le groupe formé par l'eau oxygénée, le peroxyde d'urée et les persels tels que le persulfate d'ammonium. On utilise de préférence une solution d'eau oxygénée à 6% en poids (20 volumes).

Selon le procédé de teinture généralement utilisé, le mélange obtenu est appliqué sur les cheveux, à la température ambiante ou à une température ne dépassant pas 40 °C, on le laisse poser pendant 10 à 40 minutes, et de préférence pendant 15 à 30 minutes, après quoi on rince les cheveux, on les lave au shampooing, on les rince à nouveau et on les sèche.

Un autre procédé de mise en oeuvre du précurseur de colorant d'oxydation de formule (I) conforme à l'invention, consiste à teindre les cheveux suivant un procédé en plusieurs temps, selon lequel, dans un premier temps, on applique le précurseur de colorant d'oxydation de type para au moyen d'une composition susdéfinie et dans un second temps, on applique le ou les coupleurs. L'agent oxydant est présent dans la composition appliquée dans le second temps ou bien est appliqué sur les cheveux eux-mêmes dans un troisième temps, les conditions de pose, de séchage et de lavage étant identiques à celles indiquées pour le procédé en une seule étape décrit ci-dessus.

L'invention est illustrée par les exemples d'application ci-après.

EXEMPLES D'APPLICATION

EXEMPLE A1

On prépare le mélange tinctorial suivant :

| | |
|---|---|
| - Amino-4 (β-hydroxyéthoxy)-3 phénol | 0,224 g |
| - p-phénylènediamine | 0,179 g |
| - Résorcine | 0,194 g |
| - m-aminophénol | 0,356 g |
| - méthyl-2 (β-hydroxyéthyl) amino-5 phénol | 0,276 g |
| - Nonylphénol oxyéthyléné avec 4 moles d'oxyde d'éthylène, vendu sous la dénomination CEMULSOL NP4 par RHONE POULENC | 12,00 g |
| - Nonylphénol oxyéthyléné avec 9 moles d'oxyde d'éthylène, vendu sous la dénomination CEMULSOL NP9 par RHONE POULENC | 15,00 g |
| - Alcool oléique polyglycérolé à 2 moles de glycérol | 1,50 g |
| - Alcool oléique polyglycérolé à 4 moles de glycérol | 1,50 g |
| - Propylèneglycol | 6,00 g |
| - Acide éthylènediamine tétra-acétique, vendu sous la dénomination TRILON B | 0,12 g |
| - Ammoniaque 22°Be | 11,00 g |
| - Eau | qsp 100,00 g |
| - pH = 9 | |

Au moment de l'emploi, on ajoute 10 g d'eau oxygénée à 6% en poids (20 volumes). Le mélange est appliqué pendant 20 minutes à 35°C sur des cheveux naturellement blancs à 90%. On obtient après shampooing et rinçage une coloration châtain cigare.

EXEMPLE A2

On prépare le mélange tinctorial suivant :

| | |
|---|---|
| - Amino-4 chloro-6 (β-hydroxyéthoxy)-3 phénol | 0,51 g |
| - Dichlorhydrate de diméthoxy-2,4 diamino-1,3 benzène | 0,60 g |
| - Polymère carboxyvinylique vendu sous la dénomination CARBOPOL 934 par la Société GOODRICH CHEMICALS | 3,00 g |
| - Ethanol à 96° | 11,00 g |
| - Butoxy-2 éthanol | 5,00 g |
| - Bromure de triméthyl cétyl ammonium | 2,00 g |
| - Acide éthylènediamine tétra-acétique, vendu sous la dénomination TRILON B | 0,20 g |
| - Ammoniaque 22°Be | 10,00 g |
| - Bisulfite de sodium 35°Be | 1,00 g |
| - Eau | qsp 100,00 g |
| - pH = 9 | |

Au moment de l'emploi, on ajoute 10 g d'eau oxygénée à 6% en poids. Le mélange appliqué pendant 20 minutes à 35°C sur des cheveux décolorés leur confère après shampooing et rinçage une coloration bleu mauve.

EXEMPLE A3

On prépare le mélange tinctorial suivant :

| | |
|---|---|
| - Amino-4 méthylthio-3 phénol | 0,31 g |
| - Dichlorhydrate de di(β-hydroxyéthoxy)-4,5 diamino-1,3 benzène | 0,61 g |
| - Nonylphénol oxyéthyléné à 4 moles d'oxyde d'éthylène, vendu sous la dénomination CEMULSOL NP4 par RHONE POULENC | 12,00 g |
| - Nonylphénol oxyéthyléné à 9 moles d'oxyde d'éthylène, vendu sous la dénomination CEMULSOL NP9 par RHONE POULENC | 15,00 g |
| - Alcool oléique polyglycérolé à 2 moles de glycérol | 1,50 g |
| - Alcool oléique polyglycérolé à 4 moles de glycérol | 1,50 g |
| - Propylèneglycol | 6,00 g |
| - Acide éthylène diamine tétra-acétique, vendu sous la dénomination TRILON B | 0,12 g |
| - Ammoniaque 22° Be | 11,00 g |
| - Eau | qsp 100,00 g |
| - pH = 9,5 | |

Au moment de l'emploi, on ajoute 9 g d'eau oxygénée à 6% en poids. Le mélange appliqué pendant 25 minutes à 35° C sur des cheveux naturellement blancs à 90% leur confère après shampooing et rinçage une coloration gris flanelle.

EXEMPLE A4

On prépare le mélange tinctorial suivant :

| | |
|---|---|
| - Amino-4 (β-hydroxyéthoxy)-3 phénol | 0,42 g |
| - Dichlorhydrate de (β-hydroxyéthoxy)-1 diamino-2,4 benzène | 0,60 g |
| - Alcool oléique polyglycérolé à 2 moles de glycérol | 4,50 g |
| - Alcool oléique polyglycérolé à 4 moles de glycérol | 4,50 g |
| - Oleylamine oxyéthylénée à 12 moles d'oxyde d'éthylène, vendue sous la dénomination ETHOMEEN 012 par ARMOON HESS CHEMICAL Ltd | 4,50 g |
| - Diéthanolamides de coprah vendus sous la dénomination COMPERLAN KD par HENKEL | 9,00 g |
| - Propylèneglycol | 4,00 g |
| - Butoxy-2 éthanol | 8,00 g |
| - Ethanol 96° | 6,00 g |
| - Sel pentasodique de l'acide diéthylène triamine penta-acétique, vendu sous la dénomination MASQUOL DTPA par PROTEX | 2,00 g |
| - Hydroquinone | 0,15 g |
| - Solution de bisulfite de sodium à 35° Be | 1,30 g |
| - Ammoniaque 22° Be | 10,00 g |
| - Eau | qsp 100,00 g |
| - pH = 10 | |

Au moment de l'emploi, on ajoute 100 g d'eau oxygénée à 6% en poids. Le mélange appliqué pendant 20 minutes à 30° C sur des cheveux décolorés leur confère après shampooing et rinçage une coloration gris brun clair.

EXEMPLE A5

On prépare le mélange tinctorial suivant :

| | |
|---|---|
| - Amino-4 (β-hydroxyéthoxy)-3 phénol | 0,42 g |
| - Chlorhydrate de méthylènedioxy-4,5 méthoxy-2 aniline | 0,51 g |
| - Alcool oléique polyglycérolé à 2 moles de glycérol | 4,50 g |
| - Alcool oléique polyglycérolé à 4 moles de glycérol | 4,50 g |
| - Oleylamine oxyéthylénée à 12 moles d'oxyde d'éthylène, vendue sous la dénomination ETHOMEEN 012 par ARMOON HESS CHEMICAL Ltd | 4,50 g |
| - Diéthanolamides de coprah, vendu sous la dénomination COMPERLAN KD par HENKEL | 9,00 g |
| - Propylèneglycol | 4,00 g |
| - Butoxy-2 éthanol | 8,00 g |
| - Ethanol 96° | 6,00 g |
| - Sel pentasodique de l'acide diéthylène triamine penta-acétique, vendu sous la dénomination MASQUOL DTPA par PROTEX | 2,00 g |
| - Hydroquinone | 0,15 g |
| - Solution de bisulfite de sodium à 35° Be | 1,30 g |
| - Ammoniaque 22° Be | 10,00 g |
| - Eau | qsp 100,00 g |
| - pH = 10 | |

Au moment de l'emploi, on ajoute 100 g d'eau oxygénée à 6% en poids. Le mélange appliqué pendant 20 minutes à 30°C sur des cheveux naturellement blancs à 90% leur confère après shampooing et rinçage une coloration brun olive clair.

EXEMPLE A6

On prépare le mélange tinctorial suivant :

| | |
|---|---|
| - Amino-4 méthylthio-3 phénol | 0,39 g |
| - Méthyl-2(β-hydroxyéthyl)amino-5 phénol | 0,41 g |
| - Alcool oléique polyglycérolé à 2 moles de glycérol | 4,50 g |
| - Alcool oléique polyglycérolé à 4 moles de glycérol | 4,50 g |
| - Oleylamine oxyéthylénée à 12 moles d'oxyde d'éthylène, vendue sous la dénomination ETHOMEEN 012 par ARMOON HESS CHEMICAL Ltd | 4,50 g |
| - Diéthanolamides de coprah, vendues sous la dénomination COMPERLAN KD par HENKEL | 9,00 g |
| - Propylèneglycol | 4,00 g |
| - Butoxy-2 éthanol | 8,00 g |
| - Ethanol 96° | 6,00 g |
| - Sel pentasodique de l'acide diéthylène triamine penta-acétique, vendu sous la dénomination MASQUOL DTPA par PROTEX | 2,00 g |
| - Hydroquinone | 0,15 g |
| - Solution de bisulfite de sodium à 35° Be | 1,30 g |
| - Ammoniaque 22° Be | 10,00 g |
| - Eau | qsp 100,00 g |
| - pH = 10 | |

Au moment de l'emploi, on ajoute 100 g d'eau oxygénée à 6% en poids. Le mélange appliqué pendant 20 minutes à 30°C sur des cheveux décolorés leur confère après shampooing et rinçage une coloration orangé clair.

EXEMPLE A7

On prépare le mélange tinctorial suivant :

| | | |
|---|---|---|
| - Amino-4 (ß-hydroxyéthylthio)-3 phénol | | 0,52 g |
| - Dichlorhydrate de (ß-hydroxyéthoxy)-1 diamino-2,4 benzène | | 0,67 g |
| - Alcool cétylstéarylique vendu sous la dénomination ALFOL C 16/18 par CONDEA | | 19,00 g |
| - 2-octyl dodécanol vendu sous la dénomination EUTANOL G par HENKEL | | 4,50 g |
| - Alcool cétylstéarylique à 15 moles d'oxyde d'éthylène vendu sous la dénomination MERGITAL C.S. par HENKEL | | 2,50 g |
| - Laurylsulfate d'ammonium | | 10,00 g |
| - Polymère cationique constitué par des motifs récurrents de formule : 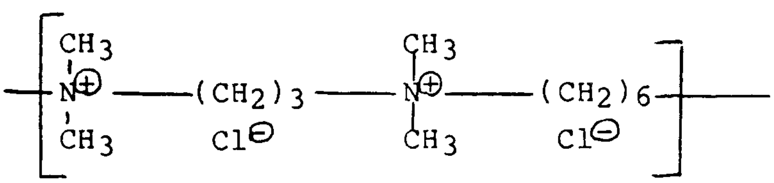 | | 4,00 g |
| - Alcool benzylique | | 2,00 g |
| - Ammoniaque 22°Be | | 11,00 ml |
| - Acide éthylène diamine tétra-acétique, vendu sous la dénomination TRILON B | | 1,00 g |
| - bisulfite de sodium à 35°Be | | 1,20 g |
| - Eau | qsp | 100,00 g |
| - pH = 10 | | |

Au moment de l'emploi, on ajoute 9 g d'eau oxygénée à 6% en poids. Le mélange appliqué 25 minutes à 35 °C sur des cheveux naturellement blancs à 90% leur confère après shampooing et rinçage une coloration châtain vison.

**Revendications**

**Revendications pour les Etats contractants suivants : AT, BE, CH, DE, FR, GB, GR, IT, LI, NL, SE**

**1.** Para-aminophénols 3-substitués de formule :

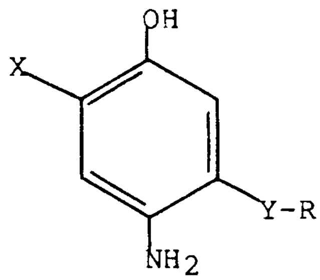

(I)

dans laquelle X représente un atome d'hydrogène ou d'halogène, Y représente un atome d'oxygène ou de soufre, R représente un radical alkyle en $C_1$-$C_4$, hydroxyalkyle en $C_1$-$C_4$, polyhydroxyalkyle en $C_3$-$C_6$ ou amino-alkyle en $C_1$-$C_4$, avec la condition que :

(i) lorsque X représente un atome d'hydrogène et Y représente un atome d'oxygène, R n'est pas méthyle ou éthyle;

(ii) lorsque X représente un atome d'hydrogène et Y représente un atome de soufre, R n'est pas méthyle;

et les sels correspondants.

**2.** Para-aminophénols 3-substitués selon la revendication 1, caractérisés par le fait que X représente un atome d'hydrogène ou d'halogène, Y représente un atome d'oxygène ou de soufre, R représente un radical alkyle en $C_1$-$C_4$, hydroxyalkyle en $C_1$-$C_4$, polyhydroxyalkyle en $C_3$-$C_6$ ou amino-alkyle en $C_1$-$C_4$, avec la condition que lorsque X représente un atome d'hydrogène, R représente un radical hydroxyalkyle en $C_1$-$C_4$, polyhydroxyalkyle en $C_3$-$C_6$ ou aminoalkyle en $C_1$-$C_4$.

**3.** Para-aminophénols substitués selon les revendications 1 ou 2, caractérisés par le fait qu'ils sont choisis dans le groupe formé par :
l'amino-4 ($\beta$-hydroxyéthoxy)-3 phénol,
l'amino-4 ($\beta$-hydroxyéthylthio)-3 phénol,
l'amino-4 chloro-6 ($\beta$-hydroxyéthoxy)-3 phénol,
l'amino-4 chloro-6 méthylthio-3 phénol,
l'amino-4 chloro-6 ($\beta$-aminoéthylthio)-3 phénol.

**4.** Para-nitrophénols substitués de formule :

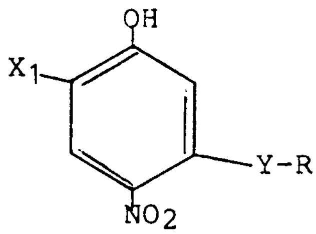

(II)

dans laquelle :

(i) Y représente un atome d'oxygène ou de soufre, $X_1$ représente un atome d'hydrogène, R représente un radical amino-alkyle en $C_{1-4}$ ou polyhydroxyalkyle en $C_3$-$C_6$, ou

(ii) Y représente un atome d'oxygène, $X_1$ représente un atome d'halogène, R représente un radical alkyle en $C_{2-4}$, polyhydroxyalkyle en $C_3$-$C_6$,hydroxyalkyle en $C_{1-4}$, amino-alkyle en $C_{1-4}$, ou

(iii) Y représente un atome de soufre, $X_1$ représente un atome d'halogène, R représente un radical alkyle en $C_{1-4}$, hydroxyalkyle en $C_{1-4}$, polyhydroxyalkyle en $C_3$-$C_6$ ou amino-alkyle en $C_{1-4}$;

et leurs sels.

**5.** Para-nitrophénols substitués selon la revendication 4, choisis dans le groupe formé par :
- le nitro-4 chloro-6 ($\beta$-hydroxyéthoxy)-3 phénol,
- le nitro-4 chloro-6 méthylthio-3 phénol,
- le nitro-4 chloro-6 ($\beta$-aminoéthylthio)-3 phénol.

**6.** Composition tinctoriale pour la teinture des fibres kératiniques et en particulier des cheveux humains, caractérisée par le fait qu'elle comprend, dans un véhicule aqueux, un ou plusieurs composés de formule (I) :

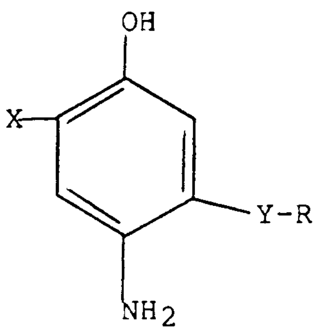

(I)

dans laquelle X représente un atome d'hydrogène ou d'halogène, Y représente un atome d'oxygène ou de soufre, R représente un radical alkyle en $C_1$-$C_4$, hydroxyalkyle en $C_1$-$C_4$, polyhydroxyalkyle en $C_3$-$C_6$ ou amino-alkyle en $C_1$-$C_4$, avec la condition que lorsque X désigne un atome d'hydrogène et Y désigne un atome d'oxygène, R n'est pas méthyle; ou un ou plusieurs sels d'un composé de formule (I).

**7.** Composition selon la revendication 6, caractérisée par le fait qu'elle comprend, dans un véhicule aqueux, un ou plusieurs composés de formule (I) dans laquelle X, Y et R ont les significations indiquées dans la revendication 6, avec la condition que lorsque X désigne un atome d'hydrogène et Y désigne un atome d'oxygène, R ne représente pas un radical alkyle.

**8.** Composition selon les revendications 6 ou 7, caractérisée par le fait qu'elle contient d'autres précurseurs de colorants par oxydation choisis parmi les para-phénylènediamines, les para-aminophénols différents de ceux de formule (I), les ortho-phénylènediamines et les ortho-aminophénols.

**9.** Composition selon l'une quelconque des revendications 6 à 8, caractérisée par le fait qu'elle comprend également un coupleur.

**10.** Composition selon la revendication 9, caractérisée par le fait que le coupleur est choisi dans le groupe formé par les méta-diphénols, les méta-aminophénols, les méta-phénylènediamines, les méta-acylaminophénols, les méta-uréidophénols, les méta-carbalcoxyaminophénols, l'alpha-naphtol, les coupleurs possédant un groupe méythylène actif tels que les composés $\beta$-cétoniques et les pyrazolones, le méthylènedioxy-3,4 phénol, le méthylènedioxy-3,4 aniline, le bromo-2 méthylènedioxy-4,5 phénol, le chloro-2 méthylènedioxy-4,5 phénol et le méthoxy-2 méthylènedioxy-4,5 aniline.

**11.** Composition selon l'une quelconque des revendications 6 à 10, caractérisée par le fait que la composition comprend également un ou plusieurs colorants directs.

**12.** Composition selon la revendication 11, caractérisée par le fait que les colorants directs sont choisis dans le groupe formé par les colorants azoiques, les colorants anthraquinoniques et les dérivés nitrés de la série benzénique.

**13.** Composition selon l'une quelconque des revendications 6 à 12, caractérisée par le fait qu'elle renferme de 0,02 à 6% en poids de composé de formule (I) par rapport au poids total de la composition.

**14.** Composition selon l'une quelconque des revendications 8 à 13, caractérisée par le fait que le poids total des précurseurs de colorants d'oxydation et des coupleurs est de 0,1 à 7% en poids du poids

total de la composition tinctoriale.

**15.** Composition selon l'une quelconque des revendications 6 à 14, caractérisée par le fait qu'elle comprend également des adjuvants choisis parmi les agents tensio-actifs anioniques, cationiques, non-ioniques, amphotères, zwittérioniques et leurs mélanges, les solvants organiques, les agents épaississants, les agents antioxydants, les agents de pénétration, les agents séquestrants, les tampons, les parfums, les agents alcalinisants.

**16.** Composition tinctoriale selon l'une quelconque des revendications 6 à 15, caractérisée par le fait que son pH est de 8 à 11.

**17.** Procédé de teinture de cheveux humains, caractérisé par le fait qu'on applique sur les cheveux pendant 10 à 40 minutes et à une température comprise entre la température ambiante et 40 °C, une quantité suffisante pour teindre des cheveux d'une composition selon l'une quelconque des revendications 6 à 16, mélangée au moment de l'emploi avec une solution oxydante, en quantité suffisante pour oxyder les précurseurs de colorants d'oxydation, après quoi on rince les cheveux, on les lave au shampooing, on les rince à nouveau et on les sèche.

**18.** Procédé de teinture des cheveux en plusieurs étapes, caractérisé par le fait que :

(i) dans une première étape, on applique sur les cheveux une quantité suffisante pour teindre les cheveux d'une composition selon l'une quelconque des revendications 6 à 8 et 10 à 16, contenant un ou plusieurs composés de formule (I), éventuellement d'autres précurseurs de colorants d'oxydation; et

(ii) dans une deuxième étape, on applique sur les cheveux une solution contenant un ou plusieurs coupleurs, l'agent oxydant étant présent dans la composition appliquée dans la deuxième étape ou bien appliqué sur les cheveux eux-mêmes dans une troisième étape, les différentes compositions étant maintenues au contact des cheveux pendant 10 à 40 minutes à une température comprise entre la température ambiante et 40 °C, après quoi on rince les cheveux, on les lave au shampooing, on les rince à nouveau et on les sèche.

**19.** Procédé de préparation d'un composé de formule (I) :

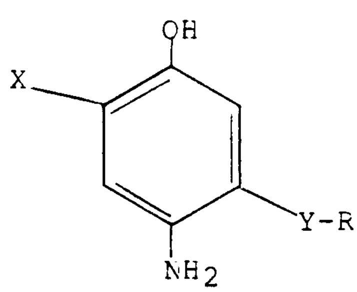

(I)

dans laquelle X représente un atome d'hydrogène ou d'halogène, Y représente un atome d'oxygène ou de soufre, R représente un radical alkyle en $C_1$-$C_4$, hydroxyalkyle en $C_1$-$C_4$, polyhydroxyalkyle en $C_3$-$C_6$ ou amino-alkyle en $C_1$-$C_4$, en plusieurs étapes :

- la première étape comprenant la réaction :

  $MOH + HYR \rightarrow H_2O + MYR$ (IV)

  où M désigne un métal alcalin ou alcalino-terreux, Y désigne un atome d'oxygène ou de soufre, et R désigne un radical alkyle en $C_1$-$C_4$, hydroxyalkyle en $C_1$-$C_4$, polyhydroxyalkyle en $C_3$-$C_6$ ou amino-alkyle en $C_1$-$C_4$;
- dans une deuxième étape, on fait réagir l'alcoolate ou thiolate de formule (IV) sur un nitrophénol de formule (III) :

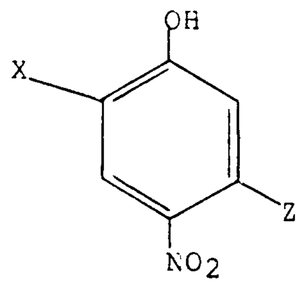

(III)

où X désigne un atome d'hydrogène ou d'halogène et Z désigne halogène pour obtenir le nitrophénol alkylé de formule (II) :

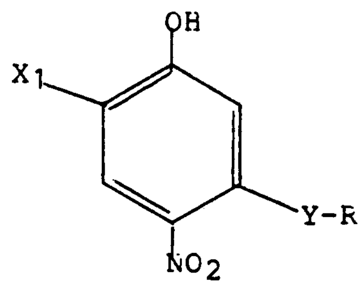

(II)

que l'on réduit dans une troisième étape pour obtenir le composé de formule (I),

la réduction pouvant être réalisée par un réducteur tel que l'hydrosulfite de sodium en milieu alcalin, à une température inférieure à 70 °C, ou bien par réduction catalytique, en milieu hydroalcoolique, sous pression d'hydrogène, en présence d'un catalyseur tel que le palladium sur charbon ou le nickel.

**20.** Procédé de préparation d'un composé de formule (I) :

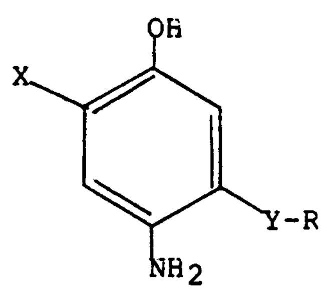

(I)

dans laquelle X désigne hydrogène, Y désigne oxygène et R désigne le radical β-hydroxyéthyle par un procédé en deux étapes, comprenant :

(a) condensation du sel de diazonium de l'acide para-sulfanilique sur le (β-hydroxyéthoxy)-3 phénol; et

(b) réduction du composé azoique obtenu en (a) par l'hydrosulfite de sodium.

**Revendications pour l'Etat contractant suivant : ES**

**1.** Procédé de préparation d'un composé de formule :

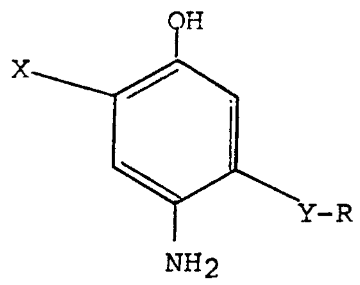

(I)

dans laquelle X représente un atome d'hydrogène ou d'halogène, Y représente un atome d'oxygène ou de soufre, R représente un radical alkyle en $C_1$-$C_4$, hydroxyalkyle en $C_1$-$C_4$, polyhydroxyalkyle en $C_3$-$C_6$ ou amino-alkyle en $C_1$-$C_4$, en plusieurs étapes :

- la première étape comprenant la réaction :

  MOH + HYR → $H_2O$ + MYR (IV)

  où M désigne un métal alcalin ou alcalino-terreux, Y désigne un atome d'oxygène ou de soufre, et R désigne un radical alkyle en $C_1$-$C_4$, hydroxyalkyle en $C_1$-$C_4$, polyhydroxyalkyle en $C_3$-$C_6$ ou amino-alkyle en $C_1$-$C_4$;
- dans une deuxième étape, on fait réagir l'alcoolate ou thiolate de formule M-Y-R (IV) sur un nitrophénol de formule :

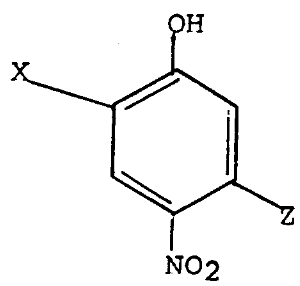

(III)

où X désigne un atome d'hydrogène ou d'halogène et Z désigne halogène pour obtenir le nitrophénol alkylé de formule :

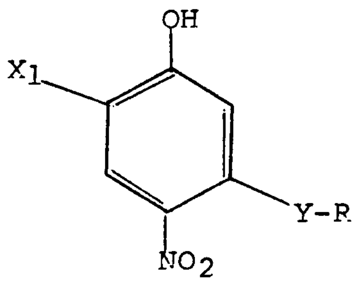

(II)

que l'on réduit dans une troisième étape pour obtenir le composé de formule (I) et, si on le désire, dans une quatrième étape, on fait réagir le composé de formule (I) sur un acide pour obtenir le sel correspondant;

la réduction pouvant être réalisée par un réducteur tel que l'hydrosulfite de sodium en milieu alcalin, à une température inférieure à 70 ° C, ou bien par réduction catalytique, en milieu hydroalcoolique, sous pression d'hydrogène, en présence d'un catalyseur tel que le palladium sur charbon ou le nickel.

2. Procédé de préparation selon la revendication 1, caractérisé par le fait que :
   (i) lorsque X représente un atome d'hydrogène et Y représente un atome d'oxygène, R n'est pas méthyle ou éthyle, et
   (ii) lorsque X représente un atome d'hydrogène et Y représente un atome de soufre, R n'est pas méthyle.

3. Procédé de préparation d'un composé de formule (I) :

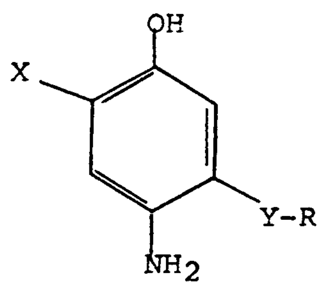

(I)

dans laquelle X désigne hydrogène, Y désigne oxygène et R désigne le radical $\beta$-hydroxyéthyle par un procédé comprenant :
   (a) condensation du sel de diazonium de l'acide para-sulfanilique sur le ($\beta$-hydroxyéthoxy)-3 phénol; et
   (b) réduction du composé azoïque obtenu en (a) par l'hydrosulfite de sodium;
   et, si on le désire, dans une troisième étape (c), on fait réagir le composé de formule (I) obtenu dans l'étape (b) sur un acide pour préparer le sel correspondant.

4. Procédé selon les revendications 1, 2 ou 3, caractérisé par le fait qu'on prépare le chlorhydrate ou le sulfate par action de l'acide chlorhydrique ou sulfurique sur le composé de formule (I).

5. Procédé de préparation selon les revendications 1 à 4, caractérisé par le fait que les composés de formule (I) sont choisis dans le groupe formé par :
   l'amino-4 ($\beta$-hydroxyéthoxy)-3 phénol,
   l'amino-4 ($\beta$-hydroxyéthylthio)-3 phénol,
   l'amino-4 chloro-6 ($\beta$-hydroxyéthoxy)-3 phénol,
   l'amino-4 chloro-6 méthylthio-3 phénol,
   l'amino-4 chloro-6 ($\beta$-aminoéthylthio)-3 phénol.

6. Procédé de préparation des para-nitro-phénols substitués de formule :

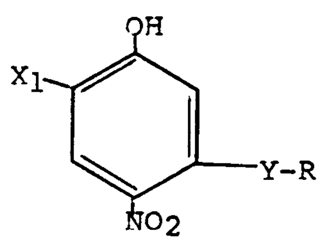

(II)

dans laquelle :
   (i) Y représente un atome d'oxygène ou de soufre, $X_1$ représente un atome d'hydrogène, R représente un radical amino-alkyle en $C_{1-4}$ ou polyhydroxyalkyle en $C_3$-$C_6$, ou
   (ii) Y représente un atome d'oxygène, $X_1$ représente un atome d'halogène, R représente un radical alkyle en $C_{2-4}$, polyhydroxyalkyle en $C_3$-$C_6$,hydroxyalkyle en $C_{1-4}$, amino-alkyle en $C_{1-4}$, ou

(iii) Y représente un atome de soufre, $X_1$ représente un atome d'halogène, R représente un radical alkyle en $C_{1-4}$, hydroxyalkyle en $C_{1-4}$, polyhydroxyalkyle en $C_3$-$C_6$ ou amino-alkyle en $C_{1-4}$; selon la réaction :

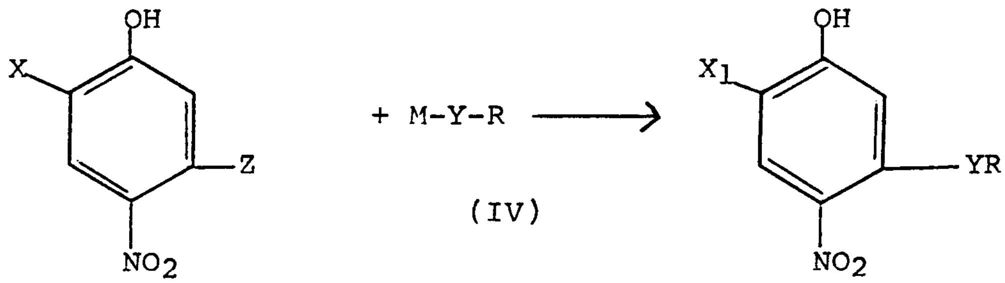


(IV)

(III) (II)

où X désigne hydrogène ou halogène; Z désigne un atome d'halogène, M désigne un métal alcalin ou alcalino-terreux, Y désigne un atome d'oxygène ou de soufre, R désigne un radical alkyle en $C_1$-$C_4$, hydroxy-alkyle en $C_1$-$C_4$, polyhydroxyalkyle en $C_3$-$C_6$ ou amino-alkyle en $C_1$-$C_4$.

**7.** Procédé de préparation selon la revendication 6, caractérisé par le fait que les paranitrophénols substitués sont choisis dans le groupe formé par :
- le nitro-4 chloro-6 ($\beta$-hydroxyéthoxy)-3 phénol,
- le nitro-4 chloro-6 méthylthio-3 phénol,
- le nitro-4 chloro-6 ($\beta$-aminoéthylthio)-3 phénol.

**8.** Procédé de préparation d'une composition tinctoriale pour la teinture des libres kératiniques et en particulier des cheveux humains, caractérisé par le fait qu'on dissout dans un véhicule aqueux, un ou plusieurs composés de formule (I) :

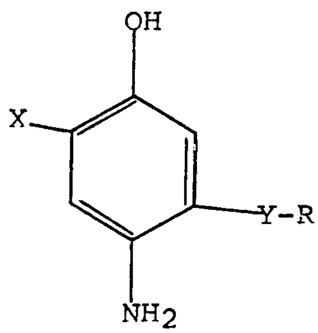

(I)

dans laquelle X représente un atome d'hydrogène ou d'halogène, Y représente un atome d'oxygène ou de soufre, R représente un radical alkyle en $C_1$-$C_4$, hydroxyalkyle en $C_1$-$C_4$, polyhydroxyalkyle en $C_3$-$C_6$ ou amino-alkyle en $C_1$-$C_4$, avec la condition que lorsque X désigne un atome d'hydrogène et Y désigne un atome d'oxygène, R n'est pas méthyle; ou un ou plusieurs sels d'un composé de formule (I).

**9.** Composition tinctoriale préparée selon la revendication 8.

**10.** Composition selon la revendication 8 ou 9, caractérisée par le fait qu'elle comprend, dans un véhicule aqueux, un ou plusieurs composés de formule (I) dans laquelle X, Y et R ont les significations indiquées dans la revendication 8, avec la condition que lorsque X désigne un atome d'hydrogène et Y désigne un atome d'oxygène, R ne représente pas un radical alkyle.

**11.** Composition selon les revendications 8, 9 ou 10, caractérisée par le fait qu'elle contient d'autres précurseurs de colorants par oxydation choisis parmi les para-phénylènediamines, les para-aminophénols différents de ceux de formule (I), les ortho-phénylènediamines et les ortho-aminophénols.

**12.** Composition selon les revendications 8 à 11, caractérisée par le fait qu'elle comprend également un coupleur.

**13.** Composition selon la revendication 12, caractérisée par le fait que le coupleur est choisi dans le groupe formé par les méta-diphénols, les méta-aminophénols, les méta-phénylènediamines, les méta-acylaminophénols, les méta-uréidophénols, les méta-carbalcoxyaminophénols, l'alpha-naphtol, les coupleurs possédant un groupe méythylène actif tels que les composés $\beta$-cétoniques et les pyrazolones, le méthylènedioxy-3,4 phénol, le méthylènedioxy-3,4 aniline, le bromo-2 méthylènedioxy-4,5 phénol, le chloro-2 méthylènedioxy-4,5 phénol et le méthoxy-2 méthylènedioxy-4,5 aniline.

**14.** Composition selon les revendications 8 à 13, caractérisée par le fait que la composition comprend également un ou plusieurs colorants directs.

**15.** Composition selon la revendication 14, caractérisée par le fait que les colorants directs sont choisis dans le groupe formé par les colorants azoiques, les colorants anthraquinoniques et les dérivés nitrés de la série benzénique.

**16.** Composition selon les revendications 8 à 15, caractérisée par le fait qu'elle renferme de 0,02 à 6% en poids de composé de formule (I) par rapport au poids total de la composition.

**17.** Composition selon les revendications 10 à 16, caractérisée par le fait que le poids total des précurseurs de colorants d'oxydation et des coupleurs est de 0,1 à 7% en poids du poids total de la composition tinctoriale.

**18.** Composition selon les revendications 8 à 17, caractérisée par le fait qu'elle comprend également des adjuvants choisis parmi les agents tensio-actifs anioniques, cationiques, non-ioniques, amphotères, zwittérioniques et leurs mélanges, les solvants organiques, les agents épaississants, les agents antioxydants, les agents de pénétration, les agents séquestrants, les tampons, les parfums, les agents alcalinisants.

**19.** Composition selon les revendications 8 à 18, caractérisée par le fait que son pH est de 8 à 11.

**20.** Procédé de teinture de cheveux humains, caractérisé par le fait qu'on applique sur les cheveux pendant 10 à 40 minutes et à une température comprise entre la température ambiante et 40 ° C, une quantité suffisante pour teindre des cheveux d'une composition tinctoriale selon les revendications 8 à 19, mélangée au moment de l'emploi avec une solution oxydante, en quantité suffisante pour oxyder les précurseurs de colorants d'oxydation, après quoi on rince les cheveux, on les lave au shampooing, on les rince à nouveau et on les sèche.

**21.** Procédé de teinture des cheveux en plusieurs étapes, caractérisé par le fait que :

(i) dans une première étape, on applique sur les cheveux une quantité suffisante pour teindre les cheveux d'une composition selon les revendications 8 à 11 et 14 à 19, contenant un ou plusieurs composés de formule (I), éventuellement d'autres précurseurs de colorants d'oxydation; et

(ii) dans une deuxième étape, on applique sur les cheveux une solution contenant un ou plusieurs coupleurs, l'agent oxydant étant présent dans la composition appliquée dans la deuxième étape ou bien appliqué sur les cheveux eux-mêmes dans une troisième étape, les différentes compositions étant maintenues au contact des cheveux pendant 10 à 40 minutes à une température comprise entre la température ambiante et 40 ° C, après quoi on rince les cheveux, on les lave au shampooing, on les rince à nouveau et on les sèche.

**Claims**

**Claims for the following Contracting States : AT, BE, CH, DE, FR, GB, GR, IT, LI, NL, SE**

**1.** 3-Substituted para-aminophenols of formula:

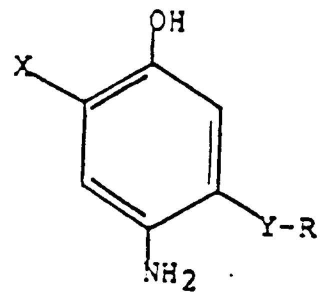

(I)

in which X denotes a hydrogen or halogen atom, Y denotes an oxygen or sulphur atom and R denotes a $C_1$-$C_4$ alkyl, $C_1$-$C_4$ hydroxyalkyl, $C_3$-$C_6$ polyhydroxyalkyl or $C_1$-$C_4$ aminoalkyl radical, with the proviso that:

(i) when X denotes a hydrogen atom and Y denotes an oxygen atom, R is not methyl or ethyl;

(ii) when X denotes a hydrogen atom and Y denotes a sulphur atom, R is not methyl;

and the corresponding salts.

**2.** 3-Substituted para-aminophenols according to Claim 1, characterised in that X denotes a hydrogen or halogen atom, Y denotes an oxygen or sulphur atom and R denotes a $C_1$-$C_4$ alkyl, $C_1$-$C_4$ hydroxyalkyl, $C_3$-$C_6$ polyhydroxyalkyl or $C_1$-$C_4$ aminoalkyl radical, with the proviso that, when X denotes a hydrogen atom, R denotes a $C_1$-$C_4$ hydroxyalkyl, $C_3$-$C_6$ polyhydroxyalkyl or $C_1$-$C_4$ aminoalkyl radical.

**3.** Substituted para-aminophenols according to Claim 1 or 2, characterised in that they are chosen from the group comprising:

4-amino-3-(β-hydroxyethoxy)phenol,
4-amino-3-(β-hydroxyethylthio)phenol,
4-amino-6-chloro-3-(β-hydroxyethoxy)phenol,
4-amino-6-chloro-3-(methylthio)phenol, and
4-amino-6-chloro-3-(β-aminoethylthio)phenol.

**4.** Substituted para-nitrophenols of formula:

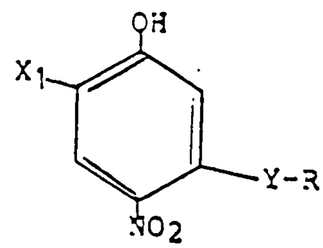

(II)

in which

(i) Y denotes an oxygen or sulphur atom, $X_1$ denotes a hydrogen atom and R denotes a $C_{1-4}$ aminoalkyl or $C_3$-$C_6$ polyhydroxyalkyl radical, or

(ii) Y denotes an oxygen atom, $X_1$ denotes a halogen atom and R denotes a $C_{2-4}$ alkyl, $C_3$-$C_6$ polyhydroxyalkyl, $C_{1-4}$ hydroxyalkyl, $C_{1-4}$ aminoalkyl radical, or

(iii) Y denotes a sulphur atom, $X_1$ denotes a halogen atom and R denotes a $C_{1-4}$ alkyl, $C_{1-4}$ hydroxyalkyl, $C_3$-$C_6$ polyhydroxyalkyl or $C_{1-4}$ aminoalkyl radical;

and their salts.

**5.** Substituted para-nitrophenols according to Claim 4, chosen from the group comprising:
4-nitro-6-chloro-3-($\beta$-hydroxyethoxy)phenol,
4-nitro-6-chloro-3-(methylthio)phenol, and
4-nitro-6-chloro-3-($\beta$-aminoethylthio)phenol.

**6.** Dyeing composition for dyeing keratinous fibres, and especially human hair, characterised in that it comprises, in an aqueous vehicle, one or more compounds of formula (I):

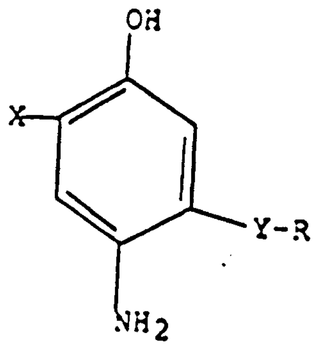

(I)

in which X denotes a hydrogen or halogen atom, Y denotes an oxygen or sulphur atom and R denotes a $C_1$-$C_4$ alkyl, $C_1$-$C_4$ hydroxyalkyl, $C_3$-$C_6$ polyhydroxyalkyl or $C_1$-$C_4$ aminoalkyl radical, with the proviso that, when X denotes a hydrogen atom and Y denotes an oxygen atom, R is not methyl; or one or more salts of a compound of formula (I).

**7.** Composition according to Claim 6, characterised in that it comprises, in an aqueous vehicle, one or more compounds of formula (I) in which X, Y and R have the meanings stated in Claim 6, with the proviso that, when X denotes a hydrogen atom and Y denotes an oxygen atom, R does not denote an alkyl radical.

**8.** Composition according to Claim 6 or 7, characterised in that it contains other oxidation dye precursors, chosen from para-phenylenediamines, para-aminophenols other than those of formula (I), ortho-phenylenediamines and ortho-aminophenols.

**9.** Composition according to any one of Claims 6 to 8, characterised in that it also comprises a coupler.

**10.** Composition according to Claim 9, characterised in that the coupler is chosen from the group comprising meta-diphenols, meta-aminophenols, meta-phenylenediamines, meta-acylaminophenols, meta-ureidophenols, meta-carbalkoxyaminophenols, alpha-naphthol, couplers possessing an active methylene group such as $\beta$-keto compounds and pyrazolones, 3,4-methylenedioxyphenol, 3,4-methylenedioxyaniline, 2-bromo-4,5-methylenedioxyphenol, 2-chloro-4,5-methylenedioxyphenol and 2-methoxy-4,5-methylenedioxyaniline.

**11.** Composition according to any one of Claims 6 to 10, characterised in that the composition also comprises one or more direct dyes.

**12.** Composition according to Claim 11, characterised in that the direct dyes are chosen from the group comprising azo dyes, anthraquinone dyes and nitro derivatives of the benzene series.

**13.** Composition according to any one of Claims 6 to 12, characterised in that it contains from 0.02 to 6% by weight of compound of formula (I) relative to the total weight of the composition.

**14.** Composition according to any one of Claims 8 to 13, characterised in that the total weight of the oxidation dye precursors and the couplers is from 0.1 to 7% by weight of the total weight of the dyeing composition.

**15.** Composition according to any one of Claims 6 to 14, characterised in that it also comprises adjuvants chosen from anionic, cationic, nonionic, amphoteric and zwitterionic surfactants, and mixtures thereof,

organic solvents, thickening agents, antioxidants, penetrating agents, sequestering agents, buffers, perfumes and alkalinising agents.

**16.** Dyeing composition according to any one of Claims 6 to 15, characterised in that its pH is from 8 to 11.

**17.** Process for dyeing human hair, characterised in that an amount, sufficient for dyeing hair, of a composition according to any one of Claims 6 to 16, mixed at the time of use with an oxidising solution in an amount sufficient for oxidising the oxidation dye precursors, is applied on the hair for 10 to 40 minutes and at a temperature between room temperature and 40 °C, after which the hair is rinsed, washed with shampoo, rinsed again and dried.

**18.** Multi-stage process for dyeing hair, characterised in that:
(i) in a first stage, an amount, sufficient for dyeing hair, of a composition according to any one of Claims 6 to 8 and 10 to 16, containing one or more compounds of formula (I) and optionally other oxidation dye precursors, is applied on the hair; and
(ii) in a second stage, a solution containing one or more couplers is applied on the hair, the oxidising agent being present in the composition applied in the second stage or alternatively applied on the hair itself in a third stage, the different compositions being maintained in contact with the hair for 10 to 40 minutes at a temperature between room temperature and 40 °C, after which the hair is rinsed, washed with shampoo, rinsed again and dried.

**19.** Multi-stage process for preparing a compound of formula (I):

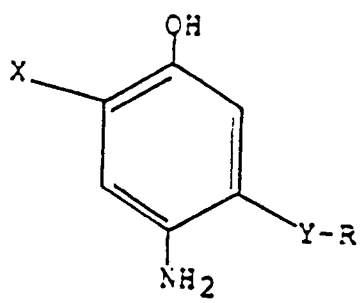

(I)

in which X denotes a hydrogen or halogen atom, Y denotes an oxygen or sulphur atom and R denotes a $C_1$-$C_4$ alkyl, $C_1$-$C_4$ hydroxyalkyl, $C_3$-$C_6$ polyhydroxyalkyl or $C_1$-$C_4$ aminoalkyl radical:

- the first stage comprising the reaction:

$$MOH + HYR \rightarrow H_2O + MYR \qquad (IV)$$

  where M denotes an alkali metal or alkaline-earth metal, Y denotes an oxygen or sulphur atom and R denotes a $C_1$-$C_4$ alkyl, $C_1$-$C_4$ hydroxyalkyl, $C_3$-$C_6$ polyhydroxyalkyl or $C_1$-$C_4$ aminoalkyl radical; and
- in a second stage, the alcoholate or thiolate of formula (IV) is reacted with a nitrophenol of formula (III):

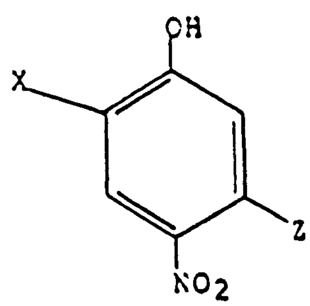

(III)

where X denotes a hydrogen or halogen atom and Z denotes halogen, to obtain the alkylated

nitrophenol of formula (II):

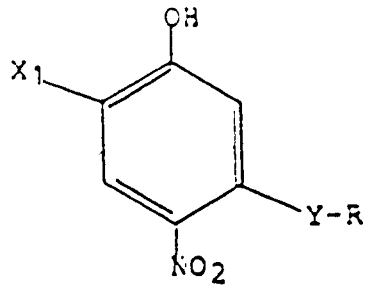

(II)

which is reduced in a third stage to obtain the compound of formula (I),

it being possible for the reduction to be carried out with a reducing agent such as sodium hydrosulphite in an alkaline medium, at a temperature below 70 °C, or alternatively by catalytic reduction, in an aqueous-alcoholic medium, under hydrogen pressure, in the presence of a catalyst such as palladium on charcoal or nickel.

**20.** Process for preparing a compound of formula (I):

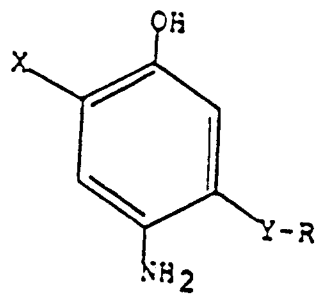

(I)

in which X denotes hydrogen, Y denotes oxygen and R denotes a $\beta$-hydroxyethyl radical, by a two-stage process comprising:

(a) condensation of the diazonium salt of para-sulphanilic acid with 3-($\beta$-hydroxyethoxy)phenol; and

(b) reduction of the azo compound obtained in (a) with sodium hydrosulphite.

**Claims for the following Contracting State : ES**

**1.** Multi-stage process for preparing a compound of formula:

(I)

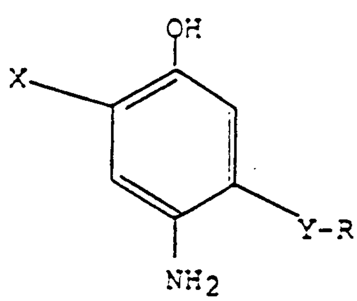

in which X denotes a hydrogen or halogen atom, Y denotes an oxygen or sulphur atom and R denotes a $C_1$-$C_4$ alkyl, $C_1$-$C_4$ hydroxyalkyl, $C_3$-$C_6$ polyhydroxyalkyl or $C_1$-$C_4$ aminoalkyl radical:

- the first stage comprising the reaction:

$$MOH + HYR \rightarrow H_2O + MYR \quad (IV)$$

where M denotes an alkali metal or alkaline-earth metal, Y denotes an oxygen or sulphur atom

and R denotes a $C_1$-$C_4$ alkyl, $C_1$-$C_4$ hydroxyalkyl, $C_3$-$C_6$ polyhydroxyalkyl or $C_1$-$C_4$ aminoalkyl radical; and

- in a second stage, the alcoholate or thiolate of formula M-Y-R (IV) is reacted with a nitrophenol of formula:

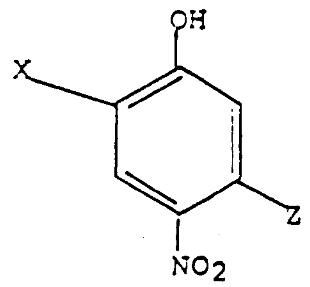

(III)

where X denotes a hydrogen or halogen atom and Z denotes halogen, to obtain the alkylated nitrophenol of formula:

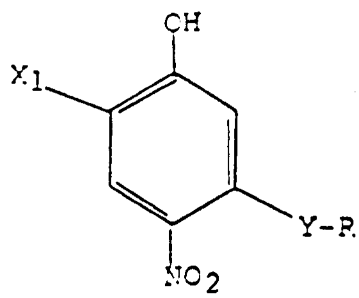

(II)

which is reduced in a third stage to obtain the compound of formula (I), and, if so desired, in a fourth stage, the compound of formula (I) is reacted with an acid to obtain the corresponding salt;

it being possible for the reduction to be carried out with a reducing agent such as sodium hydrosulphite in an alkaline medium, at a temperature below 70 °C, or alternatively by catalytic reduction, in an aqueous-alcoholic medium, under hydrogen pressure, in the presence of a catalyst such as palladium on charcoal or nickel.

**2.** Preparation process according to Claim 1, characterised in that:
(i) when X denotes a hydrogen atom and Y denotes an oxygen atom, R is not methyl or ethyl, and
(ii) when X denotes a hydrogen atom and Y denotes a sulphur atom, R is not methyl.

**3.** Process for preparing a compound of formula (I):

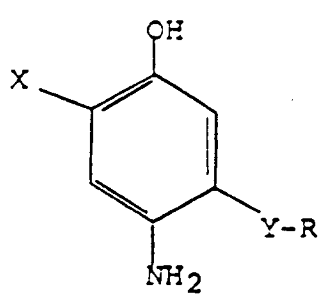

(I)

in which X denotes hydrogen, Y denotes oxygen and R denotes a $\beta$-hydroxyethyl radical, by a process comprising:

(a) condensation of the diazonium salt of para-sulphanilic acid with 3-($\beta$-hydroxyethoxy)phenol; and

(b) reduction of the azo compound obtained in (a) with sodium hydrosulphite;
and, if so desired, in a third stage (c), the compound of formula (I) obtained in stage (b) is reacted with an acid to prepare the corresponding salt.

**4.** Process according to Claim 1, 2 or 3, characterised in that the hydrochloride or the sulphate is prepared by the action of hydrochloric or sulphuric acid on the compound of formula (I).

**5.** Preparation process according to Claims 1 to 4, characterised in that the compounds of formula (I) are chosen from the group comprising:
4-amino-3-(β-hydroxyethoxy)phenol,
4-amino-3-(β-hydroxyethylthio)phenol,
4-amino-6-chloro-3-(β-hydroxyethoxy)phenol,
4-amino-6-chloro-3-(methylthio)phenol, and
4-amino-6-chloro-3-(β-aminoethylthio)phenol.

**6.** Process for preparing the substituted para-nitrophenols of formula:

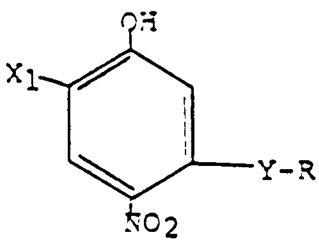

(II)

in which
(i) Y denotes an oxygen or sulphur atom, $X_1$ denotes a hydrogen atom and R denotes a $C_{1-4}$ aminoalkyl or $C_3$-$C_6$ polyhydroxyalkyl radical, or
(ii) Y denotes an oxygen atom, $X_1$ denotes a halogen atom and R denotes a $C_{2-4}$ alkyl, $C_3$-$C_6$ polyhydroxyalkyl, $C_{1-4}$ hydroxyalkyl, $C_{1-4}$ aminoalkyl radical, or
(iii) Y denotes a sulphur atom, $X_1$ denotes a halogen atom and R denotes a $C_{1-4}$ alkyl, $C_{1-4}$ hydroxyalkyl, $C_3$-$C_6$ polyhydroxyalkyl or $C_{1-4}$ aminoalkyl radical; according to the reaction:

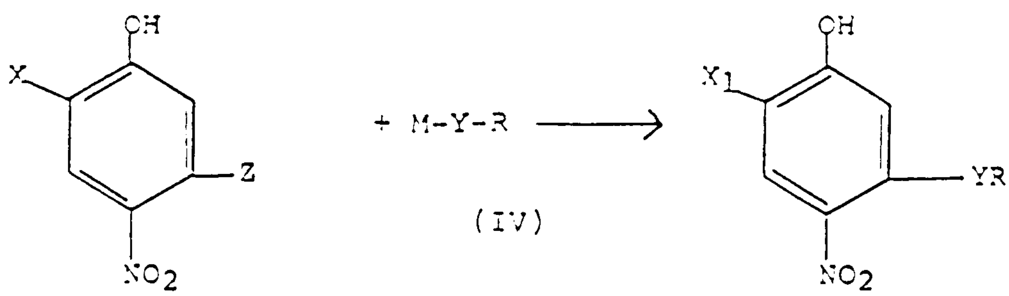

(III) (IV) (II)

where X denotes hydrogen or halogen, Z denotes a halogen atom, M denotes an alkali metal or alkaline-earth metal, Y denotes an oxygen or sulphur atom and R denotes a $C_1$-$C_4$ alkyl, $C_1$-$C_4$ hydroxyalkyl, $C_3$-$C_6$ polyhydroxyalkyl or $C_1$-$C_4$ aminoalkyl radical.

**7.** Preparation process according to Claim 6, characterised in that the substituted para-nitrophenols are chosen from the group comprising:
4-nitro-6-chloro-3-(β-hydroxyethoxy)phenol,
4-nitro-6-chloro-3-(methylthio)phenol, and
4-nitro-6-chloro-3-(β-aminoethylthio)phenol.

**8.** Process for preparing a dyeing composition for dyeing keratinous fibres, and especially human hair, characterised in that there is/are dissolved in an aqueous vehicle, one or more compounds of formula

(I):

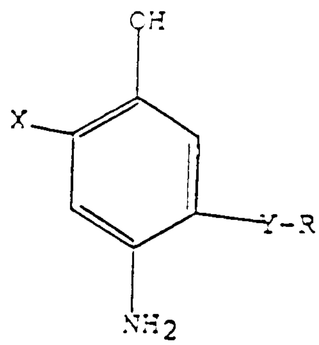

(I)

in which X denotes a hydrogen or halogen atom, Y denotes an oxygen or sulphur atom and R denotes a $C_1$-$C_4$ alkyl, $C_1$-$C_4$ hydroxyalkyl, $C_3$-$C_6$ polyhydroxyalkyl or $C_1$-$C_4$ aminoalkyl radical, with the proviso that, when X denotes a hydrogen atom and Y denotes an oxygen atom, R is not methyl; or one or more salts of a compound of formula (I).

**9.** Dyeing composition prepared according to Claim 8.

**10.** Composition according to Claim 8 or 9, characterised in that it comprises, in an aqueous vehicle, one or more compounds of formula (I) in which X, Y and R have the meanings stated in Claim 8, with the proviso that, when X denotes a hydrogen atom and Y denotes an oxygen atom, R does not denote an alkyl radical.

**11.** Composition according to Claim 8, 9 or 10, characterised in that it contains other oxidation dye precursors, chosen from para-phenylenediamines, para-aminophenols other than those of formula (I), ortho-phenylenediamines and ortho-aminophenols.

**12.** Composition according to Claims 8 to 11, characterised in that it also comprises a coupler.

**13.** Composition according to Claim 12, characterised in that the coupler is chosen from the group comprising meta-diphenols, meta-aminophenols, meta-phenylenediamines, meta-acylaminophenols, meta-ureidophenols, meta-carbalkoxyaminophenols, alpha-naphthol, couplers possessing an active methylene group such as $\beta$-keto compounds and pyrazolones, 3,4-methylenedioxyphenol, 3,4-methylenedioxyaniline, 2-bromo-4,5-methylenedioxyphenol, 2-chloro-4,5-methylenedioxyphenol and 2-methoxy-4,5-methylenedioxyaniline.

**14.** Composition according to Claims 8 to 13, characterised in that the composition also comprises one or more direct dyes.

**15.** Composition according to Claim 14, characterised in that the direct dyes are chosen from the group comprising azo dyes, anthraquinone dyes and nitro derivatives of the benzene series.

**16.** Composition according to Claims 8 to 15, characterised in that it contains from 0.02 to 6% by weight of compound of formula (I) relative to the total weight of the composition.

**17.** Composition according to Claims 10 to 16, characterised in that the total weight of the oxidation dye precursors and the couplers is from 0.1 to 7% by weight of the total weight of the dyeing composition.

**18.** Composition according to Claims 8 to 17, characterised in that it also comprises adjuvants chosen from anionic, cationic, nonionic, amphoteric and zwitterionic surfactants, and mixtures thereof, organic solvents, thickening agents, antioxidants, penetrating agents, sequestering agents, buffers, perfumes and alkalinising agents.

**19.** Composition according to Claims 8 to 18, characterised in that its pH is from 8 to 11.

**20.** Process for dyeing human hair, characterised in that an amount, sufficient for dyeing hair, of a dyeing composition according to Claims 8 to 19, mixed at the time of use with an oxidising solution in an amount sufficient for oxidising the oxidation dye precursors, is applied on the hair for 10 to 40 minutes and at a temperature between room temperature and 40 °C, after which the hair is rinsed, washed with shampoo, rinsed again and dried.

**21.** Multi-stage process for dyeing hair, characterised in that:
(i) in a first stage, an amount, sufficient for dyeing hair, of a composition according to Claims 8 to 11 and 14 to 19, containing one or more compounds of formula (I) and optionally other oxidation dye precursors, is applied on the hair; and
(ii) in a second stage, a solution containing one or more couplers is applied on the hair, the oxidising agent being present in the composition applied in the second stage or alternatively applied on the hair itself in a third stage, the different compositions being maintained in contact with the hair for 10 to 40 minutes at a temperature between room temperature and 40 °C, after which the hair is rinsed, washed with shampoo, rinsed again and dried.

**Patentansprüche**
**Patentansprüche für folgende Vertragsstaaten : AT, BE, CH, DE, FR, GB, GR, IT, LI, NL, SE**

**1.** 3-Substituierte p-Aminophenole der Formel:

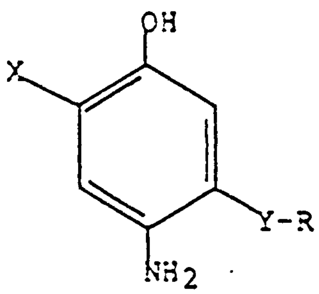

(I)

worin X ein Wasserstoff- oder Halogenatom, Y ein Sauerstoff- oder Schwefelatom und R einen $C_{1-4}$-Alkyl-, Hydroxy-$C_{1-4}$-alkyl-, Polyhydroxy-$C_{3-6}$-alkyl- oder Amino-$C_{1-4}$-alkylrest darstellen, mit der Maßgabe, daß:
(i) wenn X ein Wasserstoff- und Y ein Sauerstoffatom darstellen, R kein Methyl- oder Ethylrest ist;
(ii) wenn X ein Wasserstoff- und Y ein Schwefelatom darstellen, R kein Methylrest ist;
sowie deren Salze.

**2.** 3-Substituierte p-Aminophenole gemäß Anspruch 1, dadurch **gekennzeichnet,** daß
X ein Wasserstoff- oder Halogen-, Y ein Sauerstoffe oder Schwefelatom und R einen $C_{1-4}$-Alkyl-, Hydroxy-$C_{1-4}$-alkyl-, Polyhydroxy-$C_{3-6}$-alkyl- oder Amino-$C_{1-4}$-alkylrest darstellen, mit der Maßgabe, daß, wenn X ein Wasserstoffatom darstellt, R einen Hydroxy-$C_{1-4}$-alkyl-, Polyhydroxy-$C_{3-6}$-alkyl- oder Amino-$C_{1-4}$-alkylrest darstellt.

**3.** Substituierte p-Aminophenole gemäß der Ansprüche 1 oder 2,
dadurch **gekennzeichnet,** daß
sie ausgewählt sind aus der Gruppe aus:
4-Amino-3-(beta-hydroxyethoxy)phenol,
4-Amino-3-(beta-hydroxyethylthio)phenol,
4-Amino-6-chlor-3-(beta-hydroxyethoxy)phenol,
4-Amino-6-chlor-3-methylthiophenol,
4-Amino-6-chlor-3-(beta-aminoethylthio)phenol.

**4.** Substituierte p-Nitrophenole der Formel:

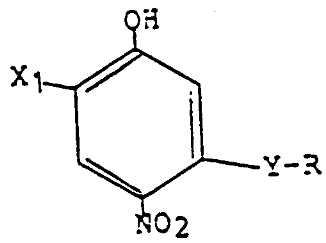

(II)

worin:

(i) Y ein Sauerstoff- oder Schwefel-, $X_1$ ein Wasserstoffatom und R einen Amino-$C_{1-4}$-alkyl- oder Polyhydroxy-$C_{3-6}$-alkylrest oder

(ii) Y ein Sauerstoff-, $X_1$ ein Halogenatom und R einen $C_{2-4}$-Alkyl-, Hydroxy-$C_{1-4}$-alkyl-, Polyhydroxy-$C_{3-6}$-alkyl oder Amino-$C_{1-4}$-alkylrest oder

(iii) Y ein Schwefel-, $X_1$ ein Halogenatom und R einen $C_{1-4}$-Alkyl-, Hydroxy-$C_{1-4}$-alkyl-, Polyhydroxy-$C_{3-6}$-alkyl- oder Amino-$C_{1-4}$-alkylrest darstellen,

sowie deren Salze.

**5.** Substituierte p-Nitrophenole gemäß Anspruch 4, ausgewählt aus der Gruppe aus:
- 4-Nitro-6-chlor-3-(beta-hydroxyethoxy)phenol,
- 4-Nitro-6-chlor-3-methylthiophenol,
- 4-Nitro-6-chlor-3-(beta-aminoethylthio)phenol.

**6.** Färbezusammensetzung zur Färbung keratinischer Fasern und insbesondere menschlicher Haare, dadurch **gekennzeichnet,** daß sie in einem wässrigen Träger eine oder mehrere Verbindungen der Formel (I):

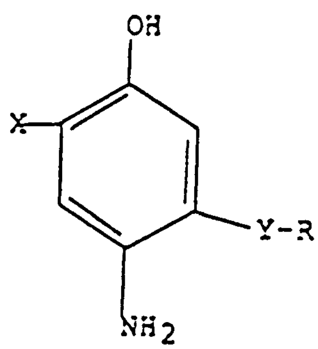

worin X ein Wasserstoff- oder Halogenatom, Y ein Sauerstoff- oder Schwefelatom und R einen $C_{1-4}$-Alkyl-, Hydroxy-$C_{1-4}$-alkyl-, Polyhydroxy-$C_{3-6}$-alkyl- oder Amino-$C_{1-4}$-alkylrest darstellen, mit der Maßgabe, daß, wenn X ein Wasserstoff- und Y ein Sauerstoffatom bedeuten, R kein Methylrest ist, oder eines oder mehrere Salze einer Verbindung der Formel (I) enthält.

**7.** Zusammensetzung gemäß Anspruch 6, dadurch **gekennzeichnet,** daß sie in einem wässrigen Träger eine oder mehrere Verbindungen der Formel (I) enthält, worin X, Y, R die in Anspruch 6 angegebenen Bedeutungen haben, mit der Maßgabe, daß, wenn X ein Wasserstoff- und Y ein Sauerstoffatom bedeuten, R keinen Alkylrest darstellt.

**8.** Zusammenetzung gemäß der Ansprüche 6 oder 7, dadurch **gekennzeichnet,** daß sie weitere Oxidationsfarbstoff-Vorstufen enthält, ausgewählt unter p-Phenylendiaminen, von denjenigen der Formel (I) verschiedenen p-Aminophenolen, o-Phenylendiaminen und o-Aminophenolen.

9. Zusammensetzung gemäß jedem Anspruch 6 bis 8,
dadurch **gekennzeichnet,** daß
sie auch einen Kuppler enthält.

10. Zusammensetzung gemäß Anspruch 9,
dadurch **gekennzeichnet,** daß
der Kuppler ausgewählt ist aus der Gruppe aus m-Diphenolen, m-Aminophenolen, m-Phenylendiaminen, m-Acylaminophenolen, m-Ureidophenolen, m-Carbalkoxyaminophenolen, alpha-Naphthol, Kupplern mit einer aktiven Methylengruppe wie beta-Ketoverbindungen und Pyrazolonen, 3,4-Methylendioxyphenol, 3,4-Methylendioxyanilin, 2-Brom-4,5-methylendioxyphenol, 2-Chlor-4,5-methylendioxyphenol und 2-Methoxy-4,5-methylendioxyanilin.

11. Zusammensetzung gemäß jedem Anspruch 6 bis 10,
dadurch **gekennzeichnet,** daß
die Zusammensetzung auch einen oder mehrere Direktfarbstoffe enthält.

12. Zusammensetzung gemäß Anspruch 11,
dadurch **gekennzeichnet,** daß
die Direktfarbstoffe ausgewählt sind aus der Gruppe aus Azo-, Anthrachinonfarbstoffen und den nitrierten Derivaten der Benzolreihe.

13. Zusammensetzung gemäß jedem Anspruch 6 bis 12,
dadurch **gekennzeichnet,** daß
sie 0,02 bis 6 Gew.% der Verbindung der Formel (I) enthält, bezogen auf Gesamtgewicht der Zusammensetzung.

14. Zusammensetzung gemäß jedem Anspruch 8 bis 13,
dadurch **gekennzeichnet,** daß
das Gesamtgewicht der Oxidationsfarbstoff-Vorstufen und Kuppler 0,1 bis 7 Gew.% des Gesamtgewichts der Färbezusammensetzung beträgt.

15. Zusammensetzung gemäß jedem Anspruch 6 bis 14,
dadurch **gekennzeichnet,** daß
sie auch Hilfstoffe enthält, ausgewählt unter anionischen, kationischen, nicht-ionischen, amphoteren, zwitterionischen oberflächenaktiven Mitteln und deren Mischungen, organischen Lösungsmitteln, Verdickern, Antioxidantien, Penetrationsmitteln, Sequestriermitteln, Tampons, Parfüms, alkalisch machenden Mitteln.

16. Färbezusammenetzung gemäß jedem Anspruch 6 bis 15,
dadurch **gekennzeichnet,** daß
ihr pH 8 bis 11 beträgt.

17. Verfahren zur Färbung menschlicher Haare,
dadurch **gekennzeichnet,** daß
man auf die Haare wahrend 10 bis 40 Minuten bei einer Temperatur von Umgebungstemperatur bis 40 °C eine zur Färbung der Haare hinreichende Menge einer Zusammenetzung gemäß jedem Anspruch 6 bis 18 aufbringt, welche zum Zeitpunkt des Gebrauchs mit einer oxidierenden Lösung vermischt wird, und zwar in einer hinreichenden Menge, um die Oxidatiaonsfarbstoff-Vorstufen zu oxidieren, worauf man die Haare spült, sie unter Schamponieren wäscht, erneut spült und trocknet.

18. Verfahren zur Färbung der Haare in mehreren Stufen,
dadurch **gekennzeichnet,** daß
man:
(i) in einer ersten Stufe auf die Haare eine zur Färbung der Haare hinreichende Menge einer Zusammensetzung gemäß jedem Anspruch 6 bis 8 und 10 bis 16, enthaltend eine oder mehrere Verbindungen der Formel (I), und gegebenenfalls weitere Oxidationsfarbstoff-Vorstufen aufbringt; und
(ii) in einer zweiten Stufe auf die Haare eine Lösung aufträgt, die einen oder mehrere Kuppler enthält, wobei das Oxidationsmittel in der in der zweiten Stufe aufgebrachten Zusammensetzung

vorhanden ist oder auch auf die Haare selbst in einer dritten Stufe aufgetragen wird, wobei die verschiedenen Zusammensetzungen in Kontakt mit den Haaren während 10 bis 40 Minuten bei einer Temperatur von Umgebungstemperatur bis 40 °C gehalten werden, worauf man die Haare spült, sie unter Schamponieren wäscht, erneut spült und trocknet.

**19.** Verfahren zur Herstellung einer Verbindung der Formel (I):

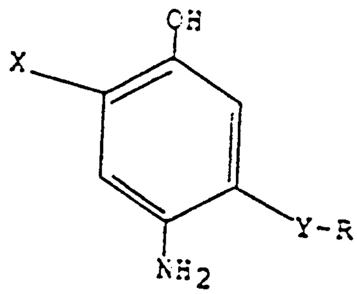

(I)

worin X ein Wasserstoff- oder Halogenatom, Y ein Sauerstoff- oder Schwefelatom und R einen $C_{1-4}$-Alkyl-, Hydroxy-$C_{1-4}$-alkyl-, Polyhydroxy-$C_{3-6}$-alkyl- oder Amino-$C_{1-4}$-alkylrest darstellen, in mehreren Stufen:

- wobei die erste Stufe die Reaktion umfaßt:

  $MOH + HYR \rightarrow H_2O + MYR$ (IV)

  worin M ein Alkali- oder Erdalkalimetall, Y ein Sauerstoffatom oder Schwefelatom und R einen $C_{1-4}$-Alkyl-, Hydroxy-$C_{1-4}$-alkyl-, Polyhydroxy-$C_{3-6}$-alkyl- oder Amino-$C_{1-4}$-alkylrest bedeuten;
- in einer zweiten Stufe läßt man das Alkoholat oder Thiolat der Formel (IV) mit einem Nitrophenol der Formel (III) reagieren:

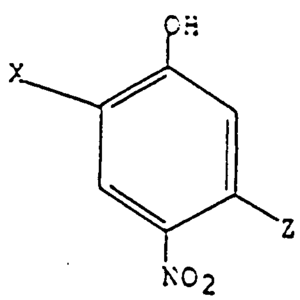

(III)

worin X ein Wasserstoff- oder Halogen- und Z ein Halogenatom bedeuten, um das alkylierte Nitrophenol der Formel (II) zu erhalten:

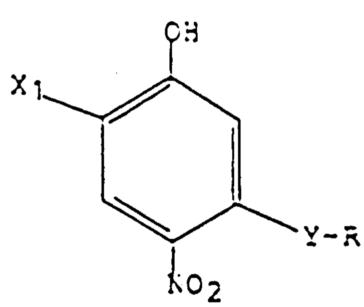

(II)

welches man in einer dritten Stufe reduziert, um die Verbindung der Formel (I) zu erhalten,
wobei die Reduktion durch ein Reduktionsmittel wie Natriumhydrogensulfit in alkalischem Milieu bei

einer Temperatur unterhalb 70 °C oder auch durch katalytische Reduktion in hydroalkoholischem Milieu unter einem Wasserstoffdruck in Gegenwart eines Katalysators wie Palladium auf Kohlenstoff oder wie Nickel durchgeführt werden kann.

**20.** Herstellungsverfahren einer Verbindung der Formel (I):

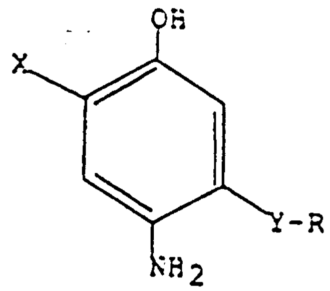

(I)

worin X ein Wasserstoff-, Y ein Sauerstoffatom und R den beta-Hydroxyethylrest bedeuten, durch ein Verfahren in zwei Stufen, wobei man:

(a) das Diazoniumsalz der p-Sulfanilsäure mit 3-(beta-Hydroxyethoxy)phenol kondensiert; und

(b) die in (a) erhaltene Azoverbindung mit Natriumhydrogensulfit reduziert.

**Patentansprüche für folgenden Vertragsstaat : ES**

**1.** Verfahren zur Herstellung einer Verbindung der Formel (I):

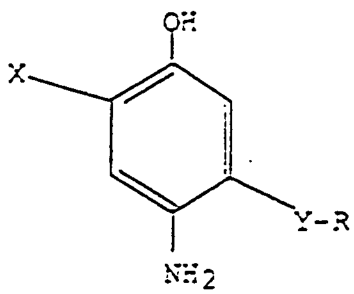

(I)

worin X ein Wasserstoff- oder Halogenatom, Y ein Sauerstoff- oder Schwefelatom und R einen $C_{1-4}$-Alkyl-, Hydroxy-$C_{1-4}$-alkyl-, Polyhydroxy-$C_{3-6}$-alkyl- oder Amino-$C_{1-4}$-alkylrest darstellen, in mehreren Stufen:

- wobei die erste Stufe die Reaktion umfaßt:

  MOH + HYR → $H_2O$ + MYR    (IV)

  worin M ein Alkali- oder Erdalkalimetall, Y ein Sauerstoffatom oder Schwefelatom und R einen $C_{1-4}$-Alkyl-, Hydroxy-$C_{1-4}$-alkyl-, Polyhydroxy-$C_{3-6}$-alkyl- oder Amino-$C_{1-4}$-alkylrest bedeuten;
- in einer zweiten Stufe läßt man das Alkoholat oder Thiolat der Formel (IV) mit einem Nitrophenol der Formel (III) reagieren:

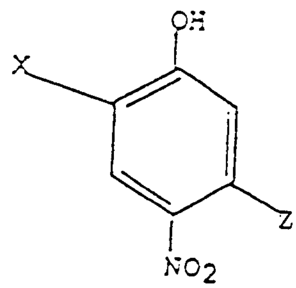

(III)

worin X ein Wasserstoff- oder Halogen- und Z ein Halogenatom bedeuten, um das alkylierte Nitrophenol der Formel (II) zu erhalten:

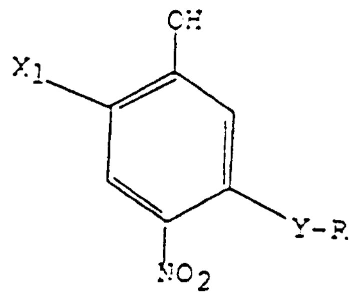

(II)

welches man in einer dritten Stufe reduziert, um die Verbindung der Formel (I) zu erhalten, wobei die Reduktion durch ein Reduktionsmittel wie Natriumhydrogensulfit in alkalischem Milieu bei einer Temperatur unterhalb 70 °C oder auch durch katalytische Reduktion in hydroalkoholischem Milieu unter einem Wasserstoffdruck in Gegenwart eines Katalysators wie Palladium auf Kohlenstoff oder wie Nickel durchgeführt werden kann.

**2.** Herstellungsverfahren gemäß Anspruch 1,
dadurch **gekennzeichnet,** daß
(i) wenn X ein Wasserstoff- und Y ein Sauerstoffatom darstellen, R kein Methyl- oder Ethylrest ist, und
(ii) wenn X ein Wasserstoff- und Y ein Schwefelatom darstellen, R kein Methylrest ist.

**3.** Herstellungsverfahren einer Verbindung der Formel (I):

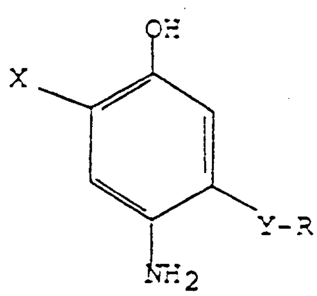

(I)

worin X ein Wasserstoff-, Y ein Sauerstoffatom und R den beta-Hydroxyethylrest bedeuten, durch ein Verfahren in zwei Stufen, wobei man:
(a) das Diazoniumsalz der p-Sulfanilsäure mit 3-(beta-Hydroxyethoxy)phenol kondensiert; und
(b) die in (a) erhaltene Azoverbindung mit Natriumhydrogensulfit reduziert;
und, falls gewünscht, in einer dritten Stufe (c) die in Stufe (b) erhaltene Verbindung der Formel (I) mit einer Säure reagieren läßt, um das entsprechende Salz herzustellen.

4. Verfahren gemäß der Ansprüche 1, 2 oder 3, dadurch **gekennzeichnet,** daß man das Hydrochlorid oder Sulfat durch Reaktion von Salz- oder Schwefelsäure mit der Verbindung der Formel (I) herstellt.

5. Herstellungsverfahren gemäß Anspruch 1 bis 4, dadurch **gekennzeichnet,** daß die Verbindungen der Formel (I) ausgewählt sind aus der Gruppe aus:
4-Amino-3-(beta-hydroxyethoxy)phenol,
4-Amino-3-(beta-hydroxyethylthio)phenol,
4-Amino-6-chlor-3-(beta-hydroxyethoxy)phenol,
4-Amino-6-chlor-3-methylthiophenol,
4-Amino-6-chlor-3-(beta-aminoethylthio)phenol.

6. Herstellungsveahren von substituierten p-Nitrophenolen der Formel:

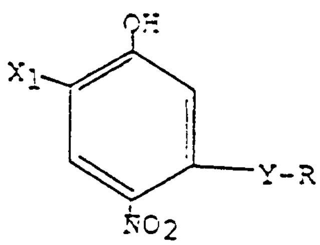

(II)

worin:
(i) Y ein Sauerstoff- oder Schwefel-, $X_1$ ein Wasserstoffatom und R einen Amino-$C_{1-4}$-alkyl- oder Polyhydroxy-$C_{3-6}$-alkylrest oder
(ii) Y ein Sauerstoff-, $X_1$ ein Halogenatom und R einen $C_{2-4}$-Alkyl-, Hydroxy-$C_{1-4}$-alkyl-, Polyhydroxy-$C_{3-6}$-alkyl oder Amino-$C_{1-4}$-alkylrest oder
(iii) Y ein Schwefel-, $X_1$ ein Halogenatom und R einen $C_{1-4}$-Alkyl-, Hydroxy-$C_{1-4}$-alkyl-, Polyhydroxy-$C_{3-6}$-alkyl- oder Amino-$C_{1-4}$-alkylrest darstellen,
gemäß der Reaktion:

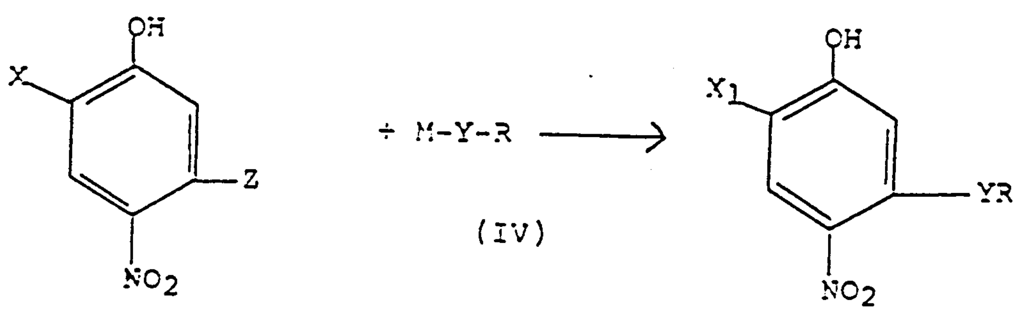


(III) (IV) (II)

worin X ein Wasserstoff- oder Halogen-, Z ein Halogenatom, M ein Alkali- oder Erdalkalimetall, Y ein Sauerstoff- oder Schwefelatom und R einen $C_{1-4}$-Alkyl-, Hydroxy-$C_{1-4}$-alkyl-, Polyhydroxy-$C_{3-6}$-alkyl- oder Amino-$C_{1-4}$-alkylrest bedeuten.

7. Herstellungsverfahren gemäß Anspruch 6, dadurch **gekennzeichnet,** daß die substituierten p-Nitrophenole ausgewählt sind aus der Gruppe aus:
- 4-Nitro-6-chlor-3-(beta-hydroxyethoxy)phenol,
- 4-Nitro-6-chlor-3-methylthiophenol,
- 4-Nitro-6-chlor-3-(beta-aminoethylthio)phenol.

**8.** Verfahren zur Herstellung einer Färbezusammensetzung zur Färbung keratinischer Fasern und insbesondere menschlicher Haare,
dadurch **gekennzeichnet,** daß
man in einem wässrigen Träger eine oder mehrere Verbindungen der Formel (I):

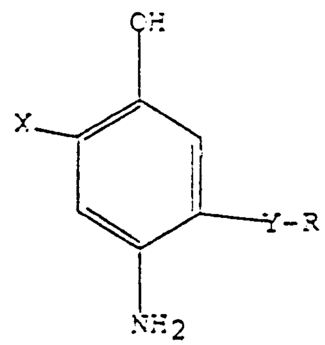

(I)

worin X ein Wasserstoff- oder Halogenatom, Y ein Sauerstoff- oder Schwefelatom und R einen $C_{1-4}$-Alkyl-, Hydroxy-$C_{1-4}$-alkyl-, Polyhydroxy-$C_{3-6}$-alkyl- oder Amino-$C_{1-4}$-alkylrest darstellen, mit der Maßgabe, daß, wenn X ein Wasserstoff- und Y ein Sauerstoffatom bedeuten, R kein Methylrest ist, oder eines oder mehrere Salze einer Verbindung der Formel (I) auflöst.

**9.** Färbezusammensetzung, hergestellt gemäß Anspruch 8.

**10.** Zusammensetzung gemäß Anspruch 8 oder 9,
dadurch **gekennzeichnet,** daß
sie in einem wässrigen Träger eine oder mehrere Verbindungen der Formel (I) enthält, worin X, Y, R die in Anspruch 8 angegebenen Bedeutungen haben, mit der Maßgabe, daß, wenn X ein Wasserstoff- und Y ein Sauerstoffatom bedeuten, R keinen Alkylrest darstellt.

**11.** Zusammenetzung gemäß der Ansprüche 8, 9 oder 10,
dadurch **gekennzeichnet,** daß
sie weitere Oxidationsfarbstoff-Vorstufen enthält, ausgewählt unter p-Phenylendiaminen, von denjenigen der Formel (I) verschiedenen p-Aminophenolen, o-Phenylendiaminen und o-Aminophenolen.

**12.** Zusammensetzung gemäß jedem Anspruch 8 bis 11,
dadurch **gekennzeichnet,** daß
sie auch einen Kuppler enthält.

**13.** Zusammensetzung gemäß Anspruch 12,
dadurch **gekennzeichnet,** daß
der Kuppler ausgewählt ist aus der Gruppe aus m-Diphenolen, m-Aminophenolen, m-Phenylendiaminen, m-Acylaminophenolen, m-Ureidophenolen, m-Carbalkoxyaminophenolen, alpha-Naphthol, Kupplern mit einer aktiven Methylengruppe wie beta-Ketoverbindungen und Pyrazolonen, 3,4-Methylendioxyphenol, 3,4-Methylendioxyanilin, 2-Brom-4,5-methylendioxyphenol, 2-Chlor-4,5-methylendioxyphenol und 2-Methoxy-4,5-methylendioxyanilin.

**14.** Zusammensetzung gemäß jedem Anspruch 8 bis 13,
dadurch **gekennzeichnet,** daß
die Zusammensetzung auch einen oder mehrere Direktfarbstoffe enthält.

**15.** Zusammensetzung gemäß Anspruch 14,
dadurch **gekennzeichnet,** daß
die Direktfarbstoffe ausgewählt sind aus der Gruppe aus Azo-, Anthrachinonfarbstoffen und den nitrierten Derivaten der Benzolreihe.

**16.** Zusammensetzung gemäß jedem Anspruch 8 bis 15,
dadurch **gekennzeichnet,** daß

sie 0,02 bis 6 Gew.% der Verbindung der Formel (I) enthält, bezogen auf Gesamtgewicht der Zusammensetzung.

**17.** Zusammensetzung gemäß jedem Anspruch 10 bis 16,
dadurch **gekennzeichnet,** daß
das Gesamtgewicht der Oxidationsfarbstoff-Vorstufen und Kuppler 0,1 bis 7 Gew.% des Gesamtgewichts der Färbezusammensetzung beträgt.

**18.** Zusammensetzung gemäß jedem Anspruch 8 bis 17,
dadurch **gekennzeichnet,** daß
sie auch Hilfstoffe enthält, ausgewählt unter anionischen, kationischen, nicht-ionischen, amphoteren, zwitterionischen oberflächenaktiven Mitteln und deren Mischungen, organischen Lösungsmitteln, Verdickern, Antioxidantien, Penetrationsmitteln, Sequestriermitteln, Tampons, Parfüms, alkalisch machenden Mitteln.

**19.** Färbezusammenetzung gemäß jedem Anspruch 8 bis 18,
dadurch **gekennzeichnet,** daß
ihr pH 8 bis 11 beträgt.

**20.** Verfahren zur Färbung menschlicher Haare,
dadurch **gekennzeichnet,** daß
man auf die Haare während 10 bis 40 Minuten bei einer Temperatur von Umgebungstemperatur bis 40 °C eine zur Färbung der Haare hinreichende Menge einer Zusammenetzung gemäß jedem Anspruch 8 bis 19 aufbringt, welche zum Zeitpunkt des Gebrauchs mit einer oxidierenden Lösung vermischt wird, und zwar in einer hinreichenden Menge, um die Oxidatiaonsfarbstoff-Vorstufen zu oxidieren, worauf man die Haare spült, sie unter Schamponieren wäscht, erneut spült und trocknet.

**21.** Verfahren zur Färbung der Haare in mehreren Stufen,
dadurch **gekennzeichnet,** daß
man:

(i) in einer ersten Stufe auf die Haare eine zur Färbung der Haare hinreichende Menge einer Zusammensetzung gemäß jedem Anspruch 8 bis 11 und 14 bis 19, enthaltend eine oder mehrere Verbindungen der Formel (I), und gegebenenfalls weitere Oxidationsfarbstoff-Vorstufen aufbringt; und
(ii) in einer zweiten Stufe auf die Haare eine Lösung aufträgt, die einen oder mehrere Kuppler enthält, wobei das Oxidationsmittel in der in der zweiten Stufe aufgebrachten Zusammensetzung vorhanden ist oder auch auf die Haare selbst in einer dritten Stufe aufgetragen wird, wobei die verschiedenen Zusammensetzungen in Kontakt mit den Haaren während 10 bis 40 Minuten bei einer Temperatur von Umgebungstemperatur bis 40 °C gehalten werden, worauf man die Haare spült, sie unter Schamponieren wäscht, erneut spült und trocknet.